(19) Europäisches Patentamt
European Patent Office
Office européen des brevets

(11) **EP 0 714 987 B1**

(12) **EUROPÄISCHE PATENTSCHRIFT**

(45) Veröffentlichungstag und Bekanntmachung des Hinweises auf die Patenterteilung:
**12.12.2001 Patentblatt 2001/50**

(51) Int Cl.[7]: **C12Q 1/68**, C07H 21/04, C12P 19/34, C12N 15/85, C07K 14/00

(21) Anmeldenummer: **95890171.2**

(22) Anmeldetag: **26.09.1995**

(54) **Verfahren zur Quantifizierung von genomischer DNA**

Method for quantifying genomic DNA

Procédé pour quantifier d'ADN génomiques

(84) Benannte Vertragsstaaten:
**AT BE CH DE DK ES FR GB IE IT LI NL SE**

(30) Priorität: **26.09.1994 AT 183094**

(43) Veröffentlichungstag der Anmeldung:
**05.06.1996 Patentblatt 1996/23**

(73) Patentinhaber: **Baxter Vaccine Aktiengesellschaft**
**1221 Wien (AT)**

(72) Erfinder:
• **Hämmerle, Thomas, Dr.**
**A-2304 Orth/Donau (AT)**
• **Falkner, Falko-Günter, Dr.**
**A-2304 Orth/Donau (AT)**
• **Kohl, Johann, Dr.**
**A-1160 Wien (AT)**
• **Himmelspach, Michele, Dr.**
**A-1100 Wien (AT)**
• **Dorner, Friedrich, Prof. Dr.**
**A-1230 Wien (AT)**

(74) Vertreter: **Weinzinger, Arnulf, Dipl.-Ing. et al**
**Patentanwälte**
**Sonn, Pawloy, Weinzinger & Köhler-Pavlik**
**Riemergasse 14**
**1010 Wien (AT)**

(56) Entgegenhaltungen:
EP-A- 0 594 959          WO-A-92/13101
WO-A-93/23573          WO-A-94/04706

• DATABASE WPI Week 8636 Derwent Publications Ltd., London, GB; AN 86-234649 XP002022980 "Detection of dna and/or rna -involves hybridising single stranded dan and/or rna labelled probe comprising repetitive sequence in dna and/or rna and detecting label. " & JP-A-61 162 752 (SEKISUI CHEM IND KK) , 23.Juli 1986
• GENE, Bd. 122, Nr. 2, 1992, Seiten 313-20, XP000471613 DIVIACCO S ET AL: "A novel procedure for quantitative polymerase chain reaction by coamplification of competitive templates"

**Beschreibung**

Die Erfindung betrifft ein Verfahren zur Quantifizierung von genomischer DNA in einer Probe, welches die Schritte

-)   Amplifizieren der in der Probe enthaltenen DNA durch ein Nukleinsäure-Amplifizierungsverfahren unter Verwendung von Primern, welche zu repetitiven genomischen Sequenzen komplementär sind, und
-)   Detektieren der erhaltenen, amplifizierten DNA umfaßt.

Der Einsatz von biotechnologisch gewonnenen Proteinen in der Medizin stellt die pharmazeutische Industrie vor neue Probleme in der Qualitätsüberprüfung ihrer Produkte. Für die Bestimmung von Verunreinigungen, die von der Zellkultur herrühren, müssen neue Nachweismethoden etabliert werden. So verlangt zum Beispiel die Welt-Gesundheits-Organisation, daß die Menge heterologer kontaminierender DNA in rekombinanten Produkten unter 100 pg pro verabreichter Dosis sein muß, wohingegen die Bestimmungen der U.S. Food and Drug Administration lediglich 10 pg DNA pro Einzeldosis akzeptieren.

Die Bestimmung von kontaminierender DNA in rekombinanten Produkten wird bisher zum größten Teil durch Hybridisierung auf Membranen gemacht. Per (Clin. Chem.35 (1989), 1859) zeigt auf, daß bis zu 5 pg DNA nachgewiesen werden können, wenn radioaktiv markierte genomische Sonden verwendet werden. Allerdings zeigt sich in einem Ringversuch, an dem sich vierzehn Labors beteiligt haben, daß die DNA-Bestimmung mittels Hybridisierung weder innerhalb eines Labors noch bei Vergleich verschiedener Labors befriedigende reproduzierbare Ergebnisse liefert (Robertson und Heath, Biologicals 20 (1992), 73).

Gilliland beschreibt eine quantitative PCR-Methode, die auf einer kompetitiven PCR-Reaktion beruht (PNAS 87 (1990), 2725). Zur Bestimmung von DNA Mengen verwendet er eine Verdünnungsreihe des internen Standard, der gleichzeitig mit der Probe amplifiziert wird. Die PCR-Reaktion wird bis zur Sättigung durchgeführt. Dies erlaubt auch die Detektion der PCR-Produkte mittels Ethidiumbromidfärbung. Die PCR-Produkte werden anschließend auf einem Gel aufgetrennt, die Kopienzahl der Probe wird mit der Kopienzahl der Standard-Verdünnungsreihe verglichen und so die Konzentration der Probe abgeschätzt. Diese Konzentrationsabschätzung ist dann exakt, wenn die Konzentration des Standards und der Probe etwa im Verhältnis 1:1 in einem Reaktionsgefäß amplifiziert wurden. Dies wiederum impliziert, daß die Bestimmung der DNA-Menge umso genauer erfolgt, je mehr Standardverdünnungen verwendet werden.

Eine Methode zur Quantifizierung von RNA wurde von Wang et al. vorgeschlagen (PNAS 86 (1989), 9717). Diese PCR-Reaktion wird in der exponentiellen Phase gestoppt. Die Autoren schaffen sich durch Amplifikation unterschiedlicher Standardkonzentrationen eine Eichkurve (externe Standardisierung). Da in der exponentiellen Reaktionsphase die Kopienanzahl der PCR-Produkte direkt proportional zur Anzahl der vorhandenen RNA-Moleküle steigt, ist diese Eichkurve eine Gerade, in der man schließlich die Konzentration einer amplifizierten Probe ablesen kann. Ein Nachteil dieser Methode ist, daß die Endkonzentration der PCR-Produkte relativ gering ist, sodaß man für die Detektion empfindliche Nachweismethoden anwenden muß. Wang et al. benutzen radioaktiv markierte Nukleotide.

Eine Verbesserung in der Detektion von geringen Mengen an PCR-Produkten gelang Porcher et al. (BioTechnique 13 (1992), 106) durch den Einsatz von Fluoreszenz-markierten Primern und der Quantifizierung der PCR-Produkte mit einem automatischen Laser-Fluoreszenz-DNA-Sequenzer.

Alle diese Methoden ermöglichen die Bestimmung geringer Mengen homologer Sequenzen, wie bestimmte Gene, virale DNA-Abschnitte oder mRNA. Eine exakte Methode zur Bestimmung von kontaminierender heterogener genomischer DNA konnte aber bisher nicht gefunden werden.

In der WO 94/12669 wird eine PCR-Methode zur Detektion von kontaminierender DNA vorgeschlagen, bei der repetitive Sequenzen des Genoms amplifiziert werden. Mit dieser Methode sollen DNA-Mengen von 0,1 pg bis zu 0,01 pg detektiert werden können. Eine geeignete Quantifizierung der chromosomalen DNA ist jedoch mit dieser Methode nicht möglich. Weder ist die Zugabe eines Standards vor der Amplifizierungsreaktion beschrieben, noch ist eine Vorgangsweise geoffenbart, wie aus der Menge an amplifizierter DNA auf die ursprünglich in der Probe enthaltene gesamte chromosomale DNA rückgeschlossen werden kann. Es wird lediglich erwähnt, daß die repetitiven Sequenzen zumindest in einer Konzentration von 1% im Genom des die Probe kontaminierenden Organismus vorhanden sein müssen. Bei der in den Beispielen beschriebenen Bestimmung kontaminierender DNA für Hamsterzellen (CHO) wird jedoch nicht angegeben, wieviele repetitive Sequenzen das CHO-Genom beinhaltet. Auch in der Literatur konnten keine Angaben dafür gefunden werden. Ohne diese Angabe ist aber eine exakte Rückrechnung auf den Gehalt an verunreinigender DNA in einer Probe prinzipiell nicht möglich, da jedenfalls eine quantitative Beziehung zwischen dem amplifizierten DNA-Stück und der gesamten chromosomalen DNA zu dieser Rückrechnung notwendig ist.

Es sei auch noch erwähnt, daß die Detektion der PCR-Produkte gemäß der WO 94/12669 mit dem Farbstoff Hoechst 32258 erfolgt, der sich in DNA einlagert. Dieser Farbstoff wird nach der PCR-Reaktion den Reaktionsprodukten zugegeben. Abgesehen davon, daß diese Analytik das Verwenden eines internen Standards ausschließt, ist diese Charakterisierung aufgrund ihrer geringen Empfindlichkeit ungeeignet für eine exakte Quantifizierung von DNA. Dies war auch gar nicht Aufgabe der WO 94/12669. In dieser Methode zur Detektion von DNA ging es lediglich darum, eine Ja/Nein-Antwort zur Frage, ob chromosomale DNA in der zu untersuchenden Probe enthalten ist, zur Verfügung zu stellen.

Für eine exakte DNA-Konzentrationsbestimmung mittels PCR ist die Zugabe eines internen Standards aber unbedingt notwendig, da sich gezeigt hat, daß die Effizienz der PCR-Reaktion oftmals von Reaktionsgefäß zu Reaktionsgefäß unterschiedlich sein kann. Dieser Effizienzunterschied kann Unterschiede in den Ergebnissen bis zu $10^5$ ergeben. Diese Fehlerquelle wird bei der Methode gemäß der WO 94/12669 völlig ignoriert, und die Reproduzierbarkeit der Methode gemäß der WO 94/12669 ist daher zweifelhaft.

Die vorliegende Erfindung stellt sich daher die Aufgabe, ein Verfahren zur Quantifizierung von chromosomaler DNA zur Verfügung zu stellen, welches eine sehr genaue quantitative Information bezüglich der Gesamtmenge an chromosomaler DNA in einer Probe ermöglicht.

Das erfindungsgemäße Verfahren der eingangs erwähnten Art ist dadurch gekennzeichnet, daß

-) vor dem Amplifizieren in an sich bekannter Weise eine gegebene Menge einer bekannten Nukleinsäure der Probe als interner Standard zugegeben wird, wobei sich die Standard-Nukleinsäure zumindest in einem zu detektierenden Merkmal von der zu quantifizierenden genomischen DNA unterscheidet, und

-) die Menge an amplifizierter genomischer DNA und die Menge an amplifizierter Standard-Nukleinsäure bestimmt werden und ausgehend von der erhaltenen Menge an Standard-Nukleinsäure die Menge der ursprünglich in der Probe vorhandenen genomischen DNA bestimmt wird.

Durch dieses Verfahren wird es erstmals möglich, den Gehalt an chromosomaler DNA in einer Probe über die Quantifizierung einer bestimmten Teilmenge der chromosomalen DNA (im vorliegenden Fall eine bestimmte repetitive Sequenz) zu bestimmen.

Unter Nukleinsäure-Amplifizierungen sind prinzipiell Verfahren zu verstehen, welche auf der von Mullis et al. (U.S. PS 4,683,195 und 4,683,202) und anderen entwickelten Technologien beruhen, beispielsweise die Polymerase-Kettenreaktion (PCR), die reverse Transkriptase PCR (RT-PCR) oder die Ligase-CR (LCR).

Die Standard-Nukleinsäure muß sich in wenigstens einem zu detektierbaren Merkmal von der zu amplifizierenden genomischen DNA unterscheiden, sie sollte aber mit Hilfe der gleichen Primer amplifiziert werden können. Als praktisch haben sich Standard-Nukleinsäuren erwiesen, die eine andere Größe als die zu amplifizierende genomische DNA oder eine von der zu amplifizierenden genomischen DNA verschiedene Restriktionsschnittstelle aufweisen. Die Standard-Nukleinsäure ist vorzugsweise eine DNA, da eine möglichst große Ähnlichkeit zwischen Standard-Nukleinsäure und der in der Probe zu quantifizierenden chromosomalen DNA bestehen sollte. Dies gilt auch für den GC-Gehalt, die Restriktionsstellen, die Sequenz, etc.. Bevorzugte Standards unterscheiden sich von der zu amplifizierenden chromosomalen DNA in 1 % bis 20 % ihrer Länge. Die genaue Sequenz der Standard-Nukleinsäure sollte natürlich bekannt sein.

Die beim Amplifizieren verwendeten Primer enthalten vorzugsweise Gruppen, welche die Nachweisgrenze der amplifizierten Nukleinsäuren erhöhen, beispielsweise fluoreszierende oder radioaktive Gruppen oder chemische Gruppen, die mit affinen Proteinen und nachgeschalteten Detektionsreaktionen detektiert werden können (z.B. Biotin-Avidin, Digoxigenin-Markierung, etc.), wobei Primer mit fluoreszierenden Gruppen besonders bevorzugt sind.

Die bevorzugte repetitive genomische Sequenz für die Analyse von DNA stellen Alu-Sequenzen oder Alu-äquivalente Sequenzen dar, wobei die Primer vorzugsweise an einen Teil der Alu-äquivalenten Konsensussequenzen, insbesondere der Alu-äquivalenten Konsensussequenzen von Säugern, insbesondere von Nagetieren und Primaten, binden.

Die Bestimmung der DNA-Mengen (unter DNA-Menge versteht man prinzipiell die Quantität an DNA; eine DNA-Menge kann z.B. in Form von Masse (mg, µg, ng, pg, ...) oder als Anzahl der Kopien eines bestimmten DNA-Moleküls angegeben werden) nach der Amplifizierung kann auf unterschiedlichste Art erfolgen, meist jedoch ist ein Schritt vorzusehen, bei welchem die amplifizierte Standard-Nukleinsäure von der amplifizierten, zu quantifizierenden genomischen DNA getrennt wird und die getrennten DNA-Mengen separat bestimmt werden. Vorzugsweise besteht dieser Trennungsschritt in einer Gelelektrophorese oder in einem chromatographischen Verfahren.

Als besonders geeignet haben sich Detektionsverfahren erwiesen, welche automatisch erfolgen und den Trennungs- und Quantifizierungsschritt kombinieren. Eine bevorzugte Ausführungsform des erfindungsgemäßen Verfahrens besteht daher darin, daß die Bestimmung der Mengen an amplifizierter Nukleinsäure unter Verwendung eines Nukleinsäure-Detektionsgerätes, vorzugsweise eines fluoreszenz-empfindlichen Nukleinsäure-Detektionsgerätes, erfolgt. Beispiele für solche Nukleinsäure-Detektionsgeräte sind automatische DNA-Sequenzer mit laserinduzierten Fluoreszenz-Meßeinrichtungen (z.B. Gene Scanner®373A der Firma Applied Biosystems) oder HPLC-Anlagen. Bei diesen Geräten ist es möglich, DNA-Moleküle voneinander zu trennen, die sich lediglich um ein bp in der Länge unterscheiden.

Ein besonderer Vorteil des Gene Scanner® ist es, unterschiedliche Fluoreszenzfarbstoffe in einer einzigen Spur unterscheiden zu können. Dies ermöglicht die gleichzeitige Aufarbeitung einer Vielzahl von Proben auf einem Gel, da alle am Gel zur Verfügung stehenden Spuren für Proben verwendet werden können. Weiters ist es möglich, eine Vielzahl von PCR-Produkten, markiert mit unterschiedlichen Fluoreszenz-Farbstoffen, in einer einzigen Bahn zu analysieren (Multiplex-PCR) und dabei genomische DNA verschiedenen Ursprungs in einer Probe nachzuweisen. Beim gleichzeitigen Nachweis von beispielsweise zwei verschiedenen Nukleinsäuren in einer Probe werden außerdem Aufwand und Kosten nahezu halbiert. Dies ist beim Einsatz des erfindungsgemäßen Verfahrens im Routinebetrieb von besonderem Vorteil. Im Gegensatz dazu kann der von Porcher et al. zur Analyse der PCR Produkte verwendete automatische Laser-Fluoreszenz-DNA-Sequenzer nur einen Fluoreszenzfarbstoff (und damit nur eine DNA) pro Spur analysieren.

Bei einer bevorzugten Ausführungsform des erfindungsgemäßen Verfahrens wird der Amplifizierungsschritt bereits in der exponentiellen Phase gestoppt.

Dadurch wird erreicht, daß das Verhältnis der Kopienanzahl des amplifizierten Standards direkt proportional zur Kopienanzahl der repetitiven Sequenz ist. Weiters kann durch Koamplifikation eines einzigen Standards die Kopienanzahl der repetitiven Sequenz festgestellt werden. In diesem Punkt ist die erfindungsgemäße Methode der viel verwendeten Methode von Gilliland et al weit überlegen, da man pro Probe lediglich einen Standard mitlaufen lassen muß, wogegen die Methode nach Gilliland um so genauer wird, desto mehr Standards in verschiedenen Verdünnungen in verschiedenen Proben verwendet werden.

Mit dem erfindungsgemäßen Verfahren wird vorzugsweise kontaminierende genomische DNA bestimmt. Diese Bestimmung ist besonders wichtig bei der Bestimmung von kontaminierender DNA in Impfstoffen oder bei der Qualitätskontrolle rekombinanter Produkte aus Zellkulturen.

Die Menge der in der Probe zu quantifizierenden genomischen DNA wird vorzugsweise mittels einer Eichgerade von der Menge an amplifizierter Standard-Nukleinsäure bestimmt.

Bisher gab es nämlich in der Literatur noch keine Hinweise, wie der Rückschluß von einer zuvor bestimmten kleineren Menge (Kopienanzahl der repetitiven Sequenzen) auf eine größere übergeordnete Menge (genomische DNA) gemacht werden soll. Es ist lediglich bekannt, daß der Anteil der repetitiven Sequenzen im Genom im Bereich von 1-10% liegt. Ein linearer Zusammenhang zwischen einer amplifizierten repetitiven Sequenz und der Gesamtmenge an genomischer DNA wurde bisher nicht geoffenbart.

Dieser Zusammenhang ist daher bevorzugterweise mit einer Eichkurve herzustellen, welche folgendermaßen ermittelt werden kann:

Verschiedene bekannte Konzentrationen einer genomischen DNA einer Spezies werden mit dem erfindungsgemäßen Verfahren in einer kompetitiven Nukleinsäure-Amplifizierungsverfahren unter Verwendung eines internen Standards amplifiziert. Das Verfahren wird in der exponentiellen Phase gestoppt, und die Mengen der amplifizierten Nukleinsäuren werden bestimmt.

Die Peak-Fläche (= Kopienzahl), die der amplifizierte interne Standard ergibt, ist dann direkt proportional zur Peak-Fläche (= Kopienzahl) der amplifizierten repetitiven Sequenz der eingesetzten genomischen DNA. Für die Eichkurve trägt man die daraus errechnete Kopienzahl der repetitiven Sequenz gegen die ursprünglich eingesetzte Menge an genomischer DNA auf. Dabei wurde überraschenderweise festgestellt, daß bei geringer DNA-Konzentration (0 bis 60 pg/ml) diese Eichkurve eine Gerade darstellt. Aus dieser Geraden kann ein Faktor errechnet werden, der schließlich für die Bestimmung von unbekannten DNA- Mengen herangezogen wird.

Nach der erfindungsgemäßen Methode wird daher die Menge an genomischer DNA in pg/ml nach der folgenden Formel berechnet:

$$m_{Probe} = A_{Probe}/A_{Standard} \times N_{Standard} \times F_1 \times D \times 1/F_2$$

worin

$A_{Probe}$ die Peak-Fläche der amplifizierten chromosomalen DNA der Probe,
$A_{Standard}$ die Peak-Fläche des amplifizierten internen Standards,
$N_{Standard}$ die eingesetzten Kopien des internen Standards,
$F_1$ das Verhältnis des Volumens des Standards zum extrahierten Volumen,
$D$ der Verdünnungsfaktor (falls die Probe vor der Extraktion verdünnt worden ist) und
$F_2$ der Umrechnungsfaktor ist, der angibt, wieviel detektierbare Kopien der repetitiven Sequenz pro pg genomischer DNA enthalten sind.

Diese Eichkurve ist charakteristisch für die verwendete genomische DNA, die eingesetzten Primer und die speziell gewählten Amplifizierungsbedingungen.

Wichtige Kriterien bei der Quantifizierung von chromosomaler DNA sind die Sensitivität und die Reproduzierbarkeit. Mit dem erfindungsgemäßen Verfahren können DNA-Mengen im Bereich von 1 pg bis 100 pg sehr genau und reproduzierbar bestimmt werden. Damit ist aber keineswegs die Sensitivitätsgrenze der Methode erreicht

Das erfindungsgemäße Verfahren eignet sich besonders gut zur Überprüfung und Qualitätskontrolle von biotechnologisch hergestellten Proteinen, wobei es keinerlei Einschränkung auf das Herstellungsverfahren dieser Proteine gibt. Beispielsweise können rekombinante Proteine, transgene Proteine oder mittels der Hybridom-Technologie gewonnene Proteine durch das erfindungsgemäße Verfahren auf ihren Gehalt an chromosomaler DNA untersucht werden. Die Multiplex-Analyse ermöglicht es, monoklonale Antikörpern, die in Hybridoma-Zellinien gezüchtet worden sind, auf kontaminierende DNA der Ursprungs-Spezien in einfacher und effizienter Weise zu untersuchen.

Die Qualitätskontrolle insbesondere bei Impfstoffen oder rekombinanten Proteinen kämpft allerdings mit Hintergrund-Problemen. So kann man beim Bestimmen von kontaminierender chromosomaler DNA bei Primaten-Zellkulturen auch die Verunreinigungen durch die Handhabung während der Produktion oder während der Aufarbeitung der Produkte durch das erfindungsgemäße Verfahren erfassen, wenn die eingesetzten Primer spezifisch für alle Primaten-Alu-Sequenzen sind. Erfolgt die Produktion von rekombinanten Proteinen allerdings in Nicht-Primaten-Zellkulturen, wie zum Beispiel CHO (Chinese Hamster Ovarienzellen), BHK (Baby Hamster Nierenzellen) oder CEC (Hühner-Embryozellen), so ist die Nachweisgrenze mit der erfindungsgemäßen Methode weit geringer, da das Problem der Verunreinigungen durch die Handhabung der Probe wegfällt. Besonders bevorzugt wird die erfindungsgemäße Quantifizierungsmethodik bei genomischer DNA aus CHO-, Vero-(Affenzellinie), BHK-, SK-Hepl-(menschliche Leberzellinie), Hybridomzellen oder CEC-Zellen angewendet, da diese Zellkulturen am gebräuchlichsten bei der Produktion von Impfstoffen, rekombinanten Proteinen oder monoklonalen Antikörpern sind.

Die Reproduzierbarkeit der erfindungsgemäßen Methode beträgt zumindest 95%. Um dies zu erreichen, muß darauf geachtet werden, daß die Effizienz der Amplifizierungsreaktion für den Standard und die Probe gleich groß ist. Die Effizienz der Amplifizierungsreaktion ist vor allem dann von Bedeutung, wenn sie in der exponentiellen Phase gestoppt wird.

Ein weiterer Aspekt der vorliegenden Erfindung betrifft daher die Verwendung des erfindungsgemäßen Verfahrens zur Überprüfung und Qualitätskontrolle von biologischen Präparaten, besonders von biotechnologisch erzeugten Präparaten, da bei diesen die Gefahr der Kontamination mit DNA besonders groß ist. Vorteilhafterweise wird das erfindungsgemäße Verfahren bei der Überprüfung und Qualitätskontrolle von HIV-Oberflächenantigen gp160, rekombinanten Blutfaktoren, Plasmaproteinen und Impfstoffen (z.B. Vakzine gegen Herpes-, Influenza- oder Tick-Borne-Encephalitis(TBE)-Viren) verwendet.

Durch die hohe Empfindlichkeit und die besonders niedrige Nachweisgrenze des erfindungsgemäßen Verfahrens können neue Qualitätskriterien bei biologischen Produkten festgestellt werden, welche durch einen äußerst geringen bzw. fehlenden Gehalt an kontaminierenden Nukleinsäuren definiert sind.

Beeinflußt wird die Effizienz der PCR-Reaktion zum Beispiel von der Bindung der Primer an Standard- und Probe-Nukleinsäuren. Deshalb werden vorzugsweise dieselben Primer für den Standard und die Probe verwendet und diese Primer sollten möglichst 100% homolog zu den Primerbindungsstellen von Standard und zu amplifizierender genomischer DNA sein. Für den synthetischen Standard stellt dies kein Problem dar, wohl aber für die zu amplifizierenden genomischen Sequenzen. Repetitive Sequenzen sind nämlich nicht notwendigerweise zu 100% homolog, sie haben oft unterschiedliche Sequenzen. Bei der Auswahl der Primer muß daher darauf geachtet werden, daß die am strengsten konservierte Nukleotidabfolge herangezogen wird. Je weniger der Primer homolog zur genomischen repetitiven Sequenz ist, desto schlechter erfolgt die Bindung des Primers und umso schlechter ist auch die Effizienz der PCR- Reaktion. Die Primer im erfindungsgemäßen Verfahren werden durch Alignment konservierter DNA-Abschnitte innerhalb der repetitiven Sequenzen in Gendatenbanken ausgesucht.

Daher betrifft die vorliegende Erfindung gemäß einem weiteren Aspekt Primer, welche im vorliegenden Verfahren zur Anwendung kommen, nämlich

```
Alu A2/2:    GCCGGGCGTAGTGGCGGGCGCCTGTAGT bp 149-176  Seq.ID 1


Alu B:       GAGACAGAGTCTCGCTCTGTCGCCCAGG bp 294-267  Seq.ID 2
```

(Numerierung nach Batzer et al. (Nucl.Acid Res. 18 (1990) 6793)

```
CR1:         ATGAGGCACTGGAACAGGTTGCCC bp 260-283     Seq.ID 3
```

```
CR1A          CAGGGCCACATCCAGCCTGG          bp 345-326          Seq ID 4
```

(Numerierung nach Stumph et al. (PNAS 81 (1984) 6667-6671). und Plasmide für die Herstellung der Standards, nämlich

pAlu-wt (bestehend aus dem bekannten pCRII-Plasmid und einem Insert an der multiplen Klonierungsstelle, welches Insert die Basenpaare (bp) 148 bis 294 der Alu-repeat-spezifischen Sequenz aus Batzer et al. enthält)

pAlu20 (abgeleitet aus pAlu-wt mit einer Deletion von 20 bp an bp 178 der Alu-repeat-spezifischen Sequenz aus Batzer et al.)

pCR1-wt (bestehend aus dem bekannten pCRII-Plasmid (Firma InVitrogen) und einem Insert an der EcoRI-Stelle des pCRII-Plasmids, welches Insert die bp 260 bis 345 der CR1-Sequenz aus Stumph et al. (1984) enthält)

pCR1+11 (abgeleitet aus dem Plasmid pCR1-wt, indem eine 11 Nukleotide lange Insertion an der bp300-Stelle eingefügt wurde)

pCR1-8 (abgeleitet aus dem Plasmid pCR1-wt, indem eine 8 Nukleotide lange Deletion an der Position 302 (gemäß Stumph et al.) vorgenommen wurde)

Die Effizienz der Amplifizierungsreaktion hängt auch von der Art des zu amplifizierenden DNA Moleküls ab. Es hat sich für das erfindungsgemäße Verfahren von Vorteil erwiesen, den internen Standard in linearisierter Form vor der Amplifizierung zuzusetzen. Dadurch werden weitere Unterschiede in der Effizienz der Reaktion ausgeglichen, die auf die unterschiedliche Form der zu amplifizierenden DNA zurückzuführen sind.

Gemäß einem weiteren Aspekt bezieht sich die vorliegende Erfindung auch auf ein Set zur Quantifizierung genomischer Nukleinsäuren in einer Probe, welches umfaßt:

- mindestens eine bekannte Nukleinsäure als internen Standard, welche sich von den zu quantifizierenden Nukleinsäuren in mindestens einem nachweisbaren Charakteristikum unterscheidet,
- Fluoreszenz-markierte Primer, die an die Standardnukleinsäure und an die zu quantifizierende Nukleinsäure binden,
- positive Kontrollen, welche bekannte Mengen an genomischer Nukleinsäure aufweisen,
- eine negative Kontrolle, welche einen von Nukleinsäuren freien Puffer aufweist,
- eine Arbeitsanleitung.

Bevorzugte Ausführungsformen des Sets gemäß der vorliegenden Erfindung sind die folgenden:

1.) Set zur Quantifizierung genomischer DNA von Primaten in einer Probe, welches umfaßt:

- als internen Standard, das Plasmid pAlu 20
- die Fluoreszenz-markierten Primer Alu A2/2 und AluB
- positive Kontrollen, die bekannte Mengen an Vero-Zell-DNA aufweisen
- eine negative Kontrolle, die einen von genomischer Nukleinsäure freien Puffer aufweist und
- eine Arbeitsanleitung.

2.) Set zur Quantifizierung genomischer Vogel-DNA in einer Probe, welches umfaßt:

- als internen Standard, die Plasmide pCR1+11 und pCR1-8
- die Fluoreszenz-markierten Primer CR1 und CR1A
- positive Kontrollen, die bekannte Mengen an Vogel-DNA aufweisen,
- eine negative Kontrolle, die einen von genomischer Nukleinsäure freien Puffer aufweist und
- eine Arbeitsanleitung.

Die Erfindung wird in den nachstehenden Beispielen und den dazugehörigen Zeichnungsfiguren, auf die sie jedoch nicht beschränkt sein soll, noch weiter erläutert. Insbesondere wird in den Beispielen gezeigt, daß das erfindungsgemäße Verfahren sich hervorragend für eine routinemäßige, schnelle und trotzdem genaue und reproduzierbare Quantifizierung von chromosomaler DNA in unterschiedlichsten Proben eignet.

Es zeigen:

Fig.1 die Eichgerade für chromosomale Affen-DNA, wobei die Menge an genomischer DNA in pg/ml gegen die Anzahl der gefundenen Kopien pro ml aufgetragen wurde;

Fig.2 die Ergebnisse der Quantifizierung von chromosomaler Affen-DNA; und

Fig.3 die Ergebnisse der Quantifizierung von chromosomaler Hühner-DNA.

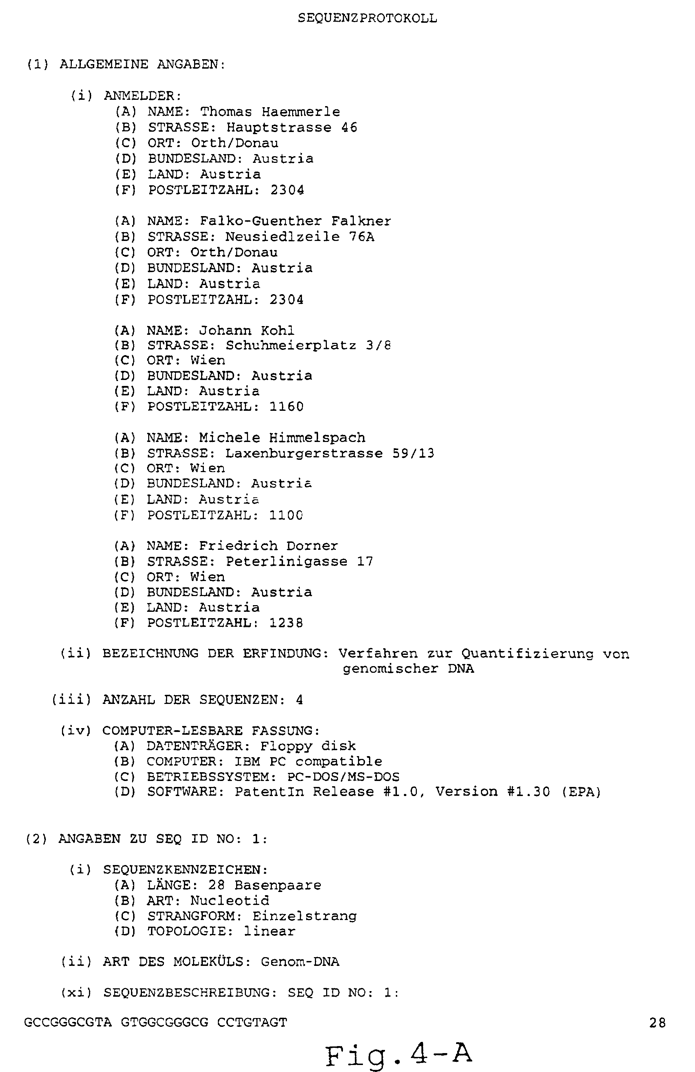

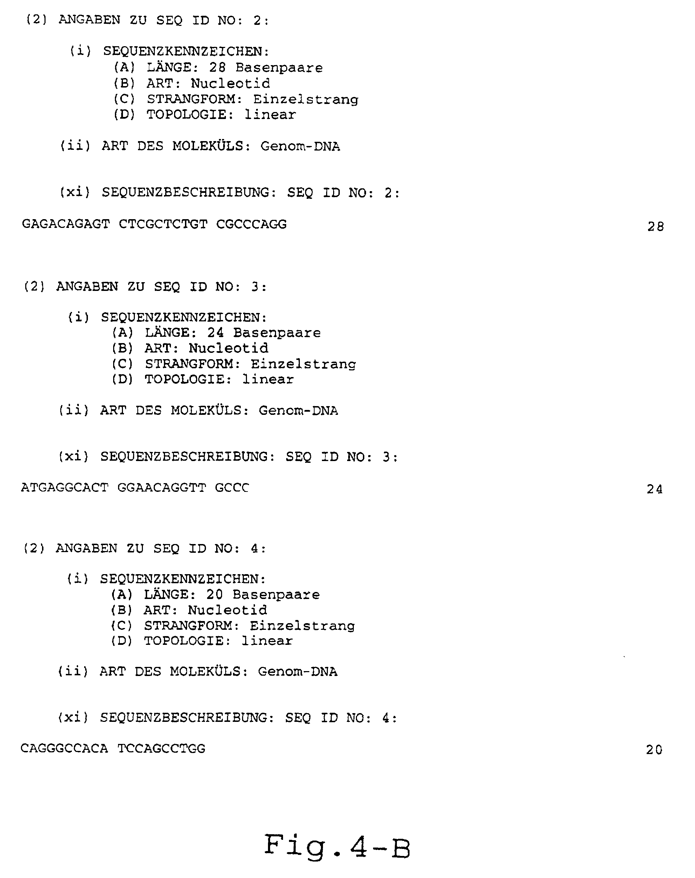

Fig.4 ist das Sequenzprotokoll.

Beispiele

1. Allgemeine Arbeitsvorschriften:

1.1. Prinzip des Verfahrens

Nukleinsäuren unterschiedlicher Herkunft werden mittels PCR unter Verwendung von Primern, welche fluoreszierende Gruppen haben, amplifiziert (Saiki et al., Science 239 (1985) 487-491). Die Analyse und die Quantifizierung der erhaltenen amplifizierten PCR-Produkte wurde mit Hilfe eines automatischen DNA-Sequenzierers mit laserinduzierter Fluoreszenz-Meßeinrichtung (373A Gene-Scanner® von Applied Biosystems) ausgeführt. Dieses Instrument ist in der Lage, die Fluoreszenz-markierten PCR-Produkte mittels einer Gelelektrophorese in einem Polyacrylamidgel unter denaturierenden Bedingungen der Größe nach aufzutrennen und deren Menge quantitativ zu bestimmen. Die Kopienanzahl bestimmter Sequenzen in der Probe wird auf Grundlage der erhaltenen Intensitäten der PCR-Produkte von chromosomaler DNA und internem Standard bestimmt. Unter Anwendung eines gegebenen Verhältnisses zwischen der Anzahl an amplifizierten repetitiven chromosomalen Sequenzen pro Standard-Masse der DNA (siehe "Erstellung der Eichgeraden") kann direkt auf die gesamte in der Probe vorhandene chromosomale DNA geschlossen werden.

1.2. Extraktion der Nukleinsäuren

500 µl einer Probe werden 5 µl 1 M TRIS/HCl pH 8,0 und 10 µl Proteinase K (Boehringer Mannheim, 20 mg/ml) sowie 20 µl 20%-ige SDS-Lösung zugesetzt. Eine bestimmte Menge an Standard-Nukleinsäure und 1 µg Hering-Sperma-DNA wird zugesetzt, und die Probe wird 1 h lang bei 56°C inkubiert. Die Probe wird nacheinander mit Phenol und Chloroform extrahiert, und 10 µl Glykogen (Boehringer Mannheim, 20 mg/ml) werden zugesetzt. Anschließend wird mit Ethanol präzipitiert, zentrifugiert, das Pellet gewaschen und schließlich in Wasser wieder gelöst.

1.3. PCR

Der PCR-Ansatz enthält in bekannter Weise ein Aliquot der extrahierten Nukleinsäure, PCR-Puffer (Boehringer Mannheim), MgCl$_2$, dNTPs, Primer, Taq-DNA-Polymerase (Boehringer Mannheim, 5,0 E/µl) und Wasser. Die PCR wird gemäß den Angaben des Herstellers von Puffer und Enzym bzw. gemäß üblicher Arbeitsvorschriften (Mullis et al., Methods in Enzymology 155 (1987), 335) in einem PCR-Apparatur (GeneAmp PCR System 9600 der Firma Perkin-Elmer) durchgeführt.

1.4. Analyse der Produkte

Für die Bestimmung und Quantifizierung der PCR-Produkte werden der PCR-Lösung 0,5 bis 1,0 µl entnommen und in einem 373A Instrument der Firma Applied Biosystems und der speziellen "gene scan" Software gemäß den Angaben des Herstellers analysiert.

2.1. Beispiel 1: Quantifizierung von genomischer Affen-DNA

Bei dieser Quantifizierung werden Primer verwendet, welche in einer hochkonservierten Region in den sogenannten "Alu repeat" Sequenzen binden und ein 146 bp-Fragment amplifizieren (Jelinek et al., Ann.Rev.Biochem. 51 (1982) 813-844), nämlich

```
Alu A2/2:   GCCGGGCGTAGTGGCGGGCGCCTGTAGT bp 149-176 Seq.ID 1
```

`Alu B:    GAGACAGAGTCTCGCTCTGTCGCCCAGG bp 294-267 Seq.ID 2`

(Numerierung nach Batzer et al.). Die Primer wurden unter Verwendung der Phosphoamidit-Chemie auf einem DNA Synthesizer hergestellt (Applied Biosystems 394 DNA Synthesizer).

Das Standard-Plasmid pAlu20 ist abgeleitet aus dem Plasmid pAlu-wt, welches aus dem bekannten pCRII-Plasmid (Firma InVitrogen) und einem Insert an der multiplen Klonierungsstelle des pCRII-Plasmids besteht, welches Insert die bp 148 bis 294 der Alu-repeat-spezifischen Sequenz aus Batzer et al. enthält.

In pAlu20 wurden die bp 178 bis 197 deletiert. Das Plasmid wurde gereinigt (QUIAGEN-Verfahren), die Konzentration durch spektroskopische Messung bei 260 nm bestimmt, mit EcoRI geschnitten und in einem 10mM TRIS/HCl pH 8/0,1 mM EDTA-Puffer verdünnt (Sambrook et al. Molecular Cloning, Second Edition, Cold Spring Harbor Lab Press, Cold Spring Harbor (1989)).

Die Länge der PCR-Produkte von Standard und Wildtyp-DNA betragen daher 126 und 146 bp.

2.1: Erstellung der Eichgeraden mit Affen DNA

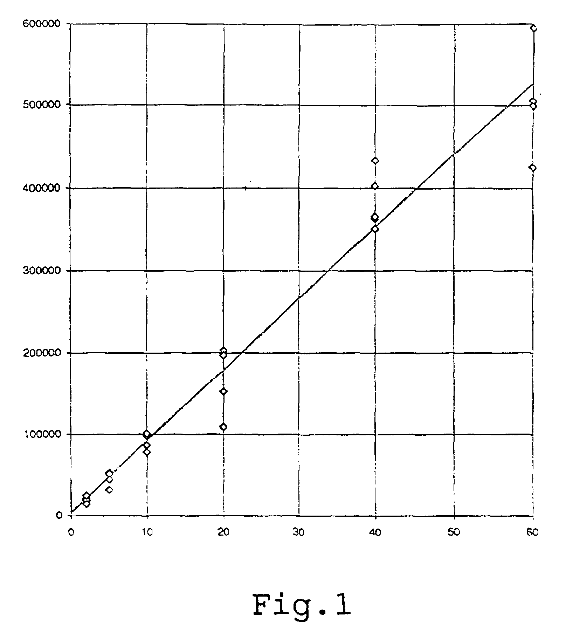

Zur Bestimmung der Anzahl Alu-Kopien pro pg genomischer Affen-DNA wurden jeweils 3 Kalibrierungsläufe durchgeführt (blank, 2 pg, 5 pg, 10 pg, 20 pg, 40 pg, 60 pg, 100 pg DNA/ml). Alle ermittelten Daten (6 PCR-Reaktionen pro Konzentrationsstufe) sind in Tabelle 1 zusammengefaßt und die Regressionsgerade ist in Fig.1 dargestellt.

TABELLE 1

| DNA | KG | RG | MW | SD | VK% |
|---|---|---|---|---|---|
| 0 | | 4533.92996 | | | |
| 2 | 21599,4238 | 21974,0922 | | | |
| 2 | 18983,3919 | 21974,0922 | 20099,8091 | 3822,30746 | 19,0166357 |
| 2 | 19900,3524 | 21974,0922 | | | |
| 2 | 25246,6524 | 21974,0922 | | | |
| 2 | 14769,225 | 21974,0922 | | | |
| 5 | 44883,8779 | 48134,3354 | | | |
| 5 | 52631,2571 | 48134,3354 | | | |
| 5 | 51109,4418 | 48134,3354 | 45055,9297 | 8108,004 | 17,9954205 |
| 5 | 32068,2966 | 48134,3354 | | | |
| 5 | 44586,7749 | 48134,3354 | | | |
| 10 | 97555,2485 | 91734,7409 | | | |
| 10 | 101015,717 | 91734,7409 | 90598.5793 | 10754.6015 | 11,8706073 |
| 10 | 86335,9144 | 91734,7409 | | | |
| 10 | 77487,4372 | 91734,7409 | | | |
| 20 | 108834,009 | 178935,552 | | | |
| 20 | 199305,642 | 178935,552 | | | |
| 20 | 203203,788 | 178935,552 | 172039,16 | 41076,2263 | 23,876091 |
| 20 | 197098,228 | 178935,552 | | | |
| 20 | 151754,131 | 178935,552 | | | |
| 40 | 363341,143 | 353337,174 | | | |
| 40 | 402578,42 | 353337,174 | | | |
| 40 | 433036,566 | 353337,174 | 383303,318 | 33743,5526 | 8,8033552 |
| 40 | 351420,48 | 353337,174 | | | |
| 40 | 366139,983 | 353337,174 | | | |

TABELLE 1   (fortgesetzt)

| DNA | KG | RG | MW | SD | VK% |
|---|---|---|---|---|---|
| 60 | 594815,223 | 527738,796 | | | |
| 60 | 505612,823 | 527738.796 | 506228.628 | 69570,4812 | 13,7428975 |
| 60 | 424808.212 | 527738,796 | | | |
| 60 | 499678 254 | 527738,796 | | | |
| DNA: DNA in pg/ml | | | | | |
| KG: Kopien gefunden | | | | | |
| RG: Regression | | | | | |
| MW: Mittelwert | | | | | |
| SD: Standardabweichung | | | | | |
| VK%: Variationskoeffizient | | | | | |

Die Berechnung der Daten erfolgte über folgende Beziehungen:

$$\text{Fläche}_{\text{Probe}} : \text{Fläche}_{\text{pAlu-20}} = x$$

$$x - (\text{Fläche}_{\text{Blank}} : \text{Fläche}_{\text{pAlu-20}}) = y \ (\text{korrigiertes Flächenverhältnis})$$

y multipliziert mit 100 000 = Kopienanzahl in 500 µl Probe = z (500 µl wurden extrahiert, 100 000 Kopien des Standardplasmids wurden zugegeben)
z multipliziert mit 2 = Kopienanzahl pro ml

Nach der Berechnung der Regressionsgeraden (y = 4533,9 + 8720,1 x) ergab sich ein durchschnittlicher Wert von 9200 Kopien pro pg genomischer Affen-DNA.

2.2. Quantifizierung von chromosomaler Affen-DNA (Vero-Zellen)

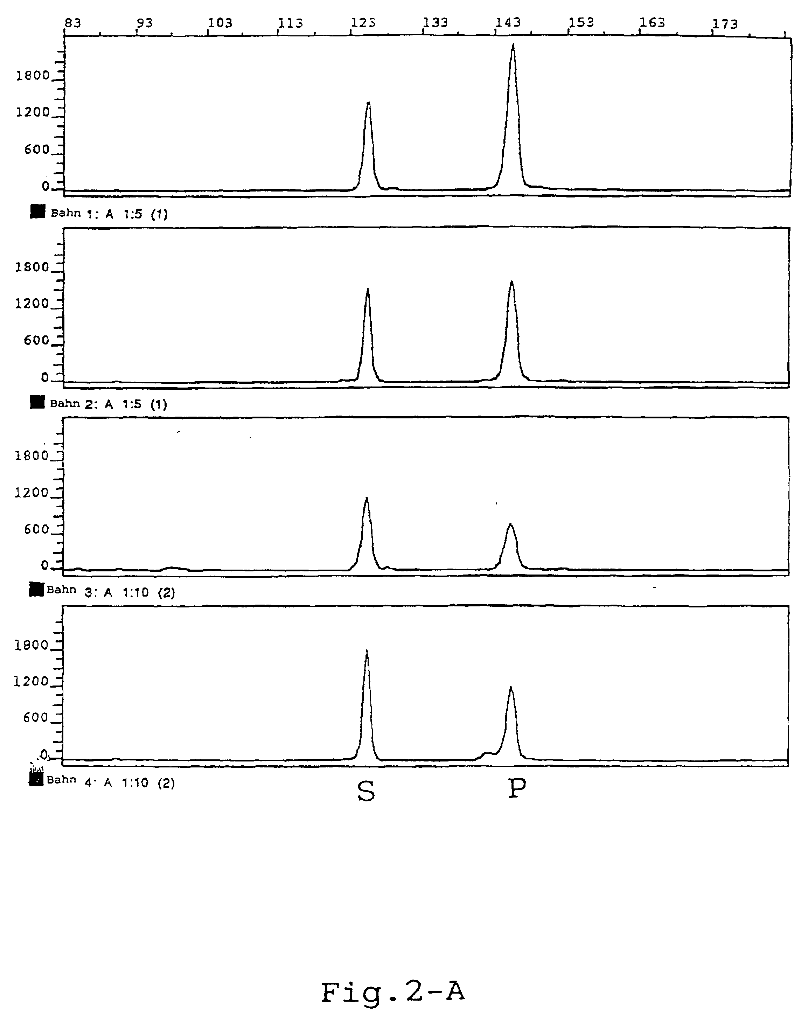

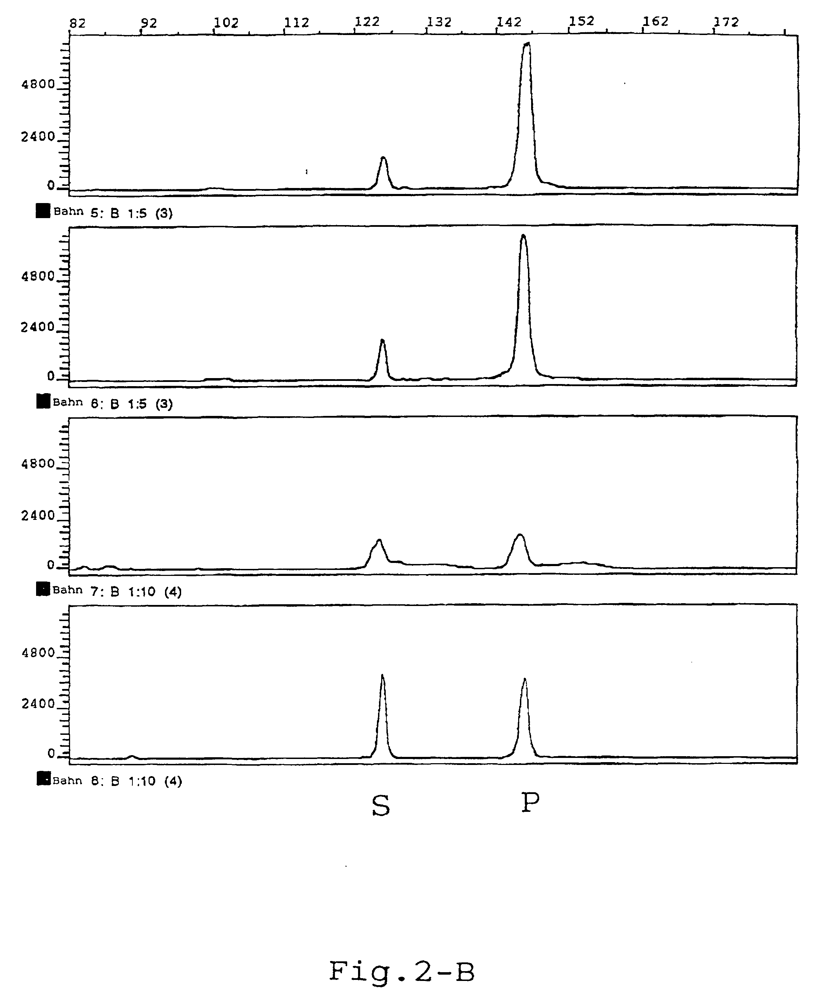

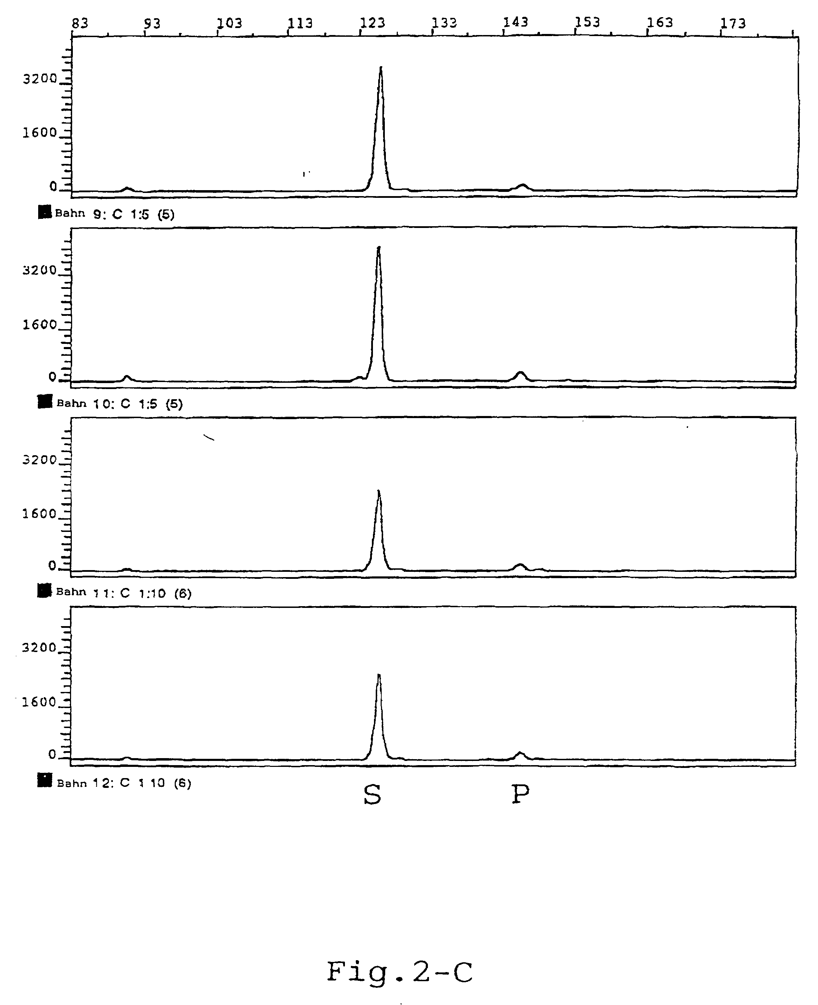

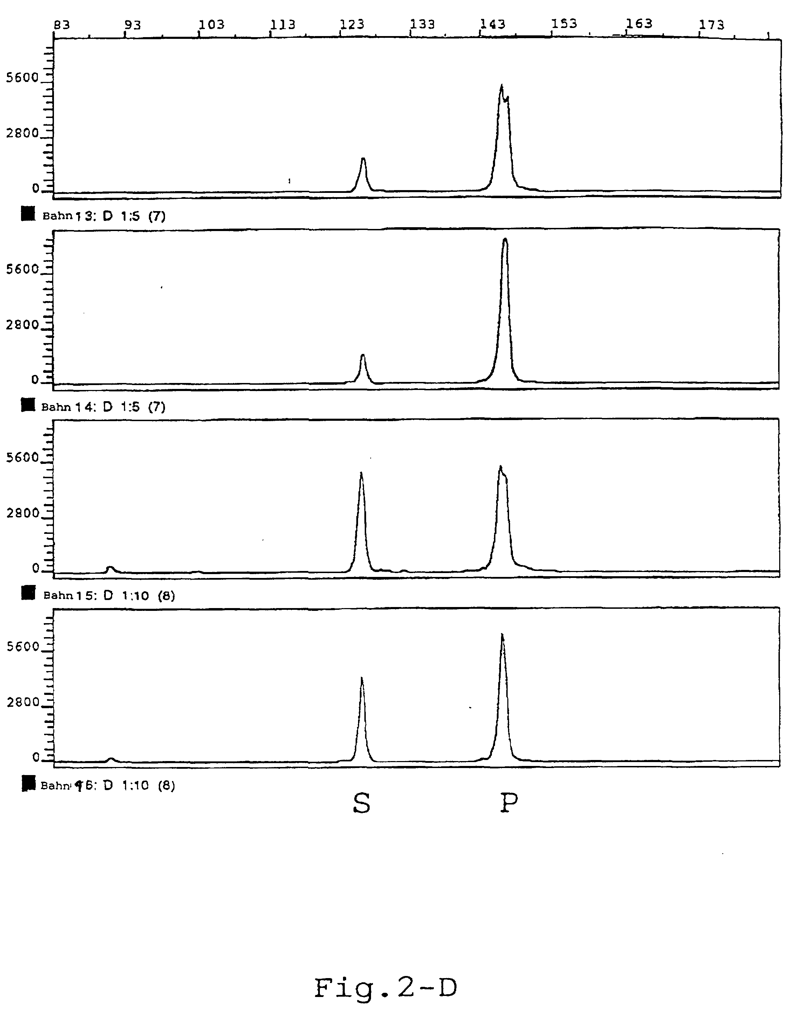

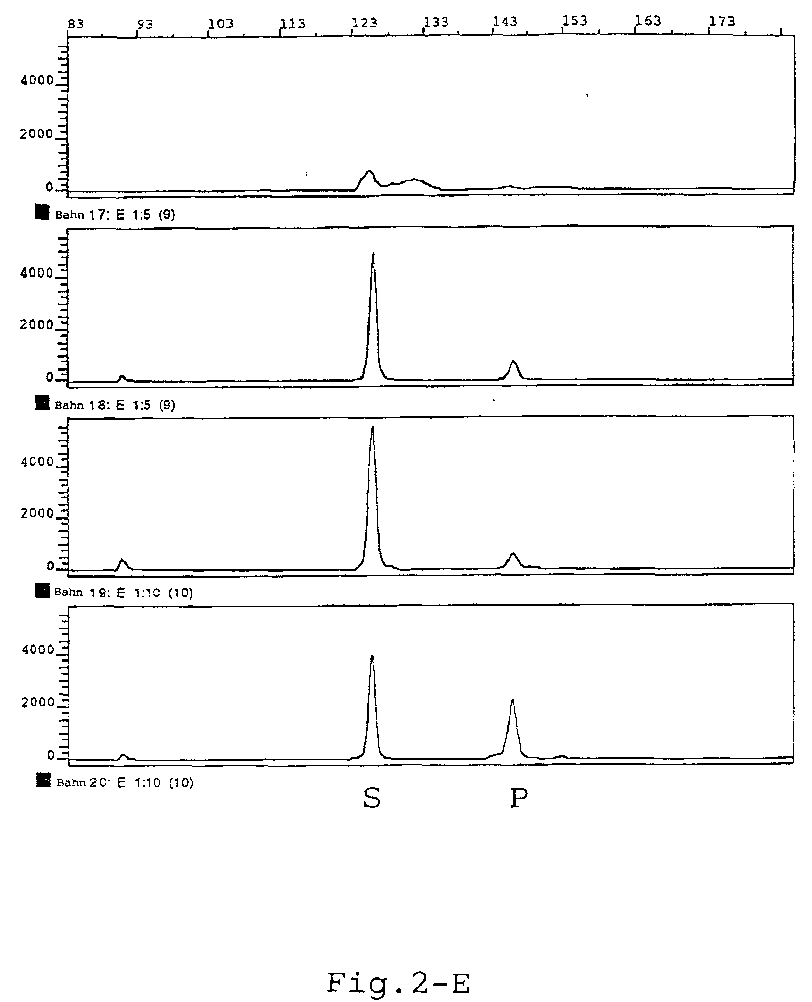

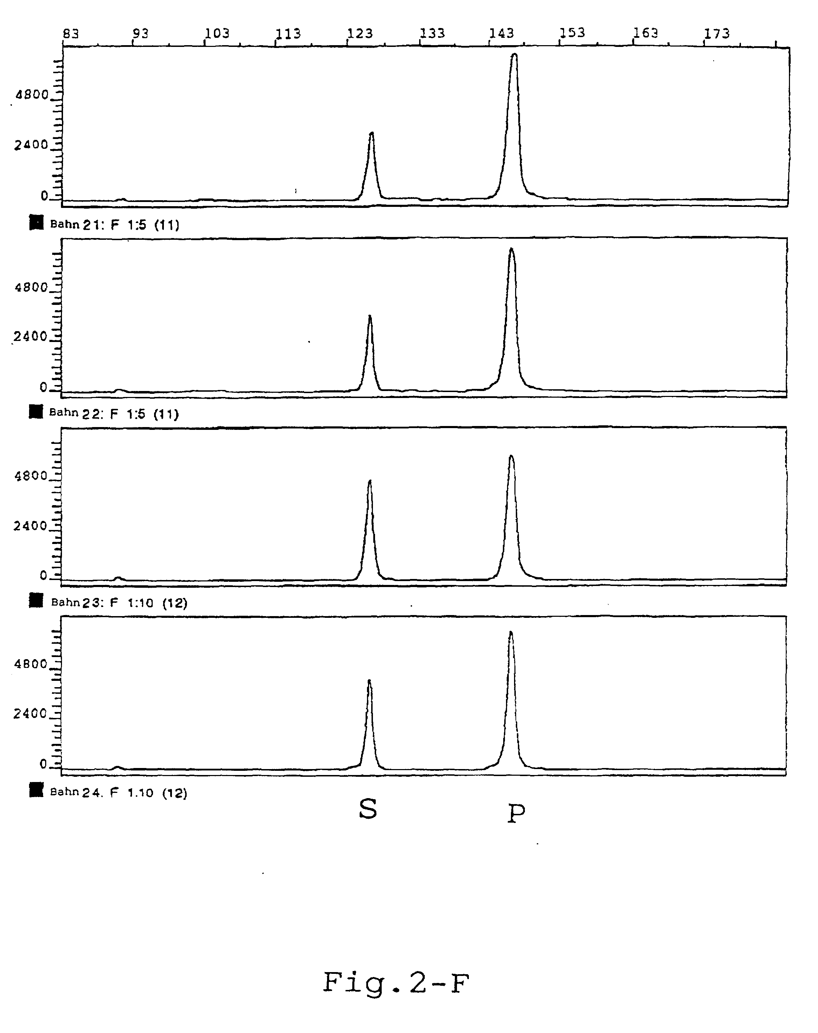

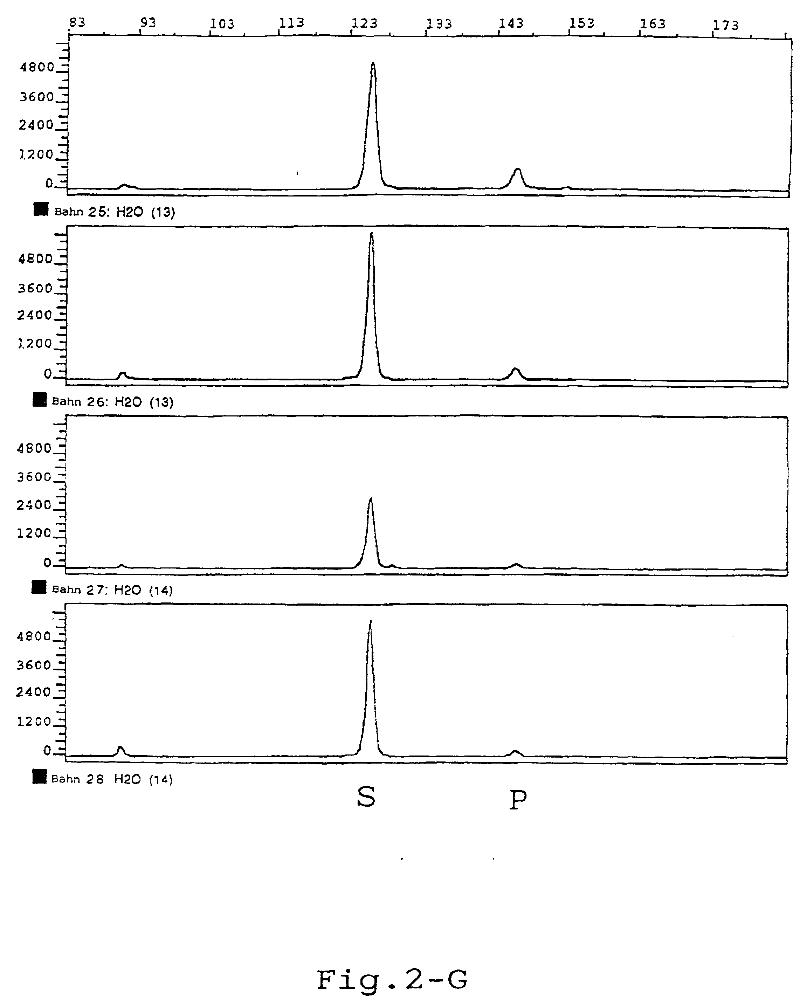

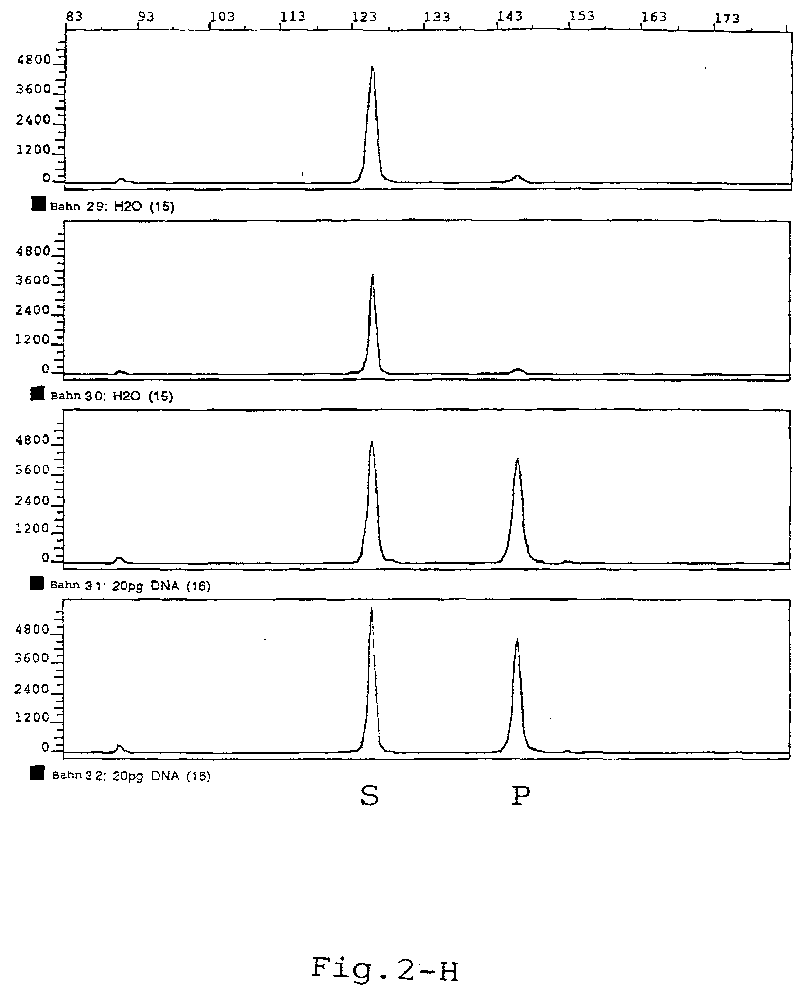

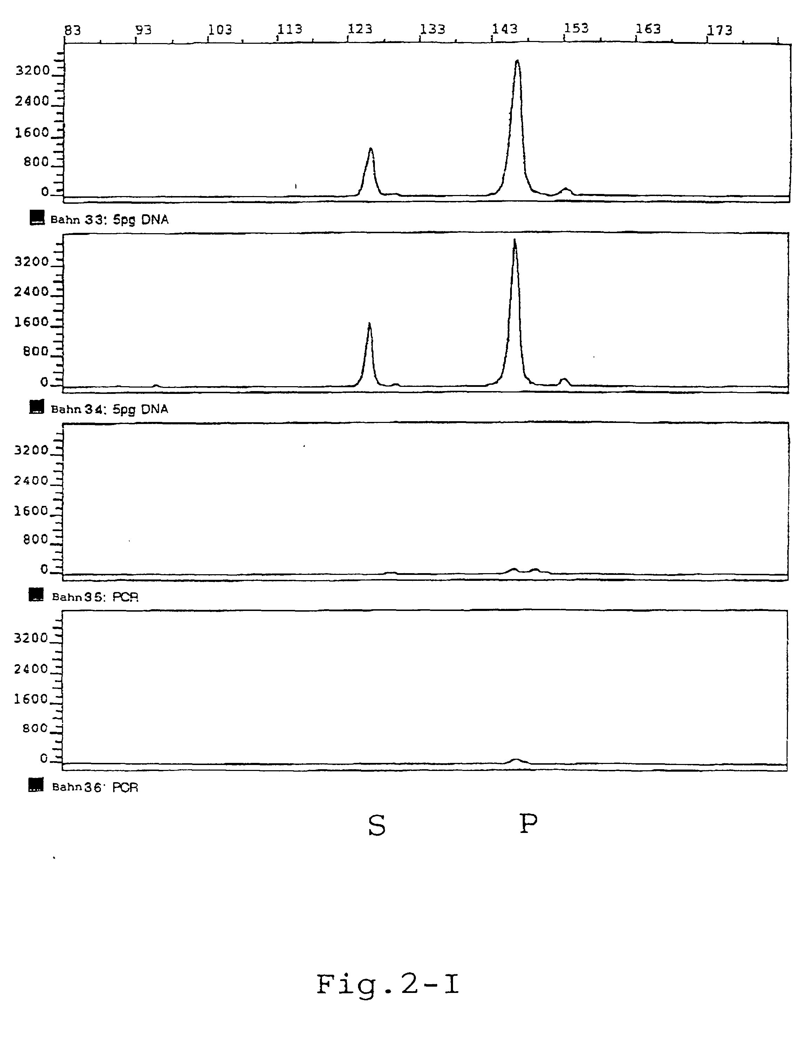

Vorstufen von Impfstoff-Chargen, die nach Barrett et al. (AIDS Research and Human Retroviruses 5 (1989), 159-171) hergestellt wurden, wurden mittels DNA-Extraktion und quantitativer PCR mit Hilfe der Primer Alu A2/2 und AluB und einem internen Standard (pAlu20), der von denselben Primern erkannt wird, jedoch ein in der Größe verschiedenes PCR-Produkt liefert, untersucht. Die Ergebnisse der Untersuchungen sind in Tabelle 2 und Fig.2-A bis 2-I dargestellt.
Tabelle 2 gibt Art, Umfang und Meßwerte der gemessenen Proben wieder. In Spalte 1 ist die Kopienzahl des Standardplasmids pAlu20 angegeben; Spalte 2 gibt die Verdünnungsstufe der Probe an, wobei der Wert 1 einer unverdünnten Probe gleichkommt; Spalte 1 gibt das extrahierte Volumen an Probe an; Spalten 4 und 5 bezeichnen die Art der Probe (Chargen-Nummer der gp160-Präparation; Puffer und Verdünnung des Standards); Spalte 6 gibt die Meßwerte in pg Vero Gesamt-DNA pro ml Probe an und Spalte 7 die Mittelwerte dieser Messungen; Spalte 8 bezeichnet die Nummer der Bahn, auf der die Probe analysiert worden ist (siehe auch: Fig.2); Spalten 9 und 10 geben die Fläche des gemessenen Standardpeaks bzw. des zu messenden Peaks an chromosomaler DNA an. Die erste Zeile ist eine Kontrollzeile.

## TABELLE 2

| Probe | 0,5 | Vol. | 100000 | Wert 1 | Wert 2 | Nr. | Wert 3 | Wert 4 |
|---|---|---|---|---|---|---|---|---|
| 311594 1:4 A 1:5 (1) | 0,5 | 5 | 100000 | 241 | 198,5 | 265,01 | 12958 | 271170 |
| 311594 1:4 A 1:5 (1) | 0,5 | 5 | 100000 | 186 | | 266,02 | 12328 | 18095 |
| 311594 1:4 A 1:10 (2) | 0,5 | 10 | 100000 | 187 | | 265,03 | 11749 | 10007 |
| 311594 1:4 A 1:10 (2) | 0,5 | 10 | 100000 | 200 | | 265,04 | 14171 | 12041 |
| 311594 B 1:5 (3) | 0,5 | 5 | 100000 | 429 | 430,5 | 265,05 | 13484 | 49634 |
| 311594 B 1:5 (3) | 0,5 | 5 | 100000 | 704 | | 265,06 | 14788 | 80535 |
| 311594 D 1:10 (4) | 0,5 | 10 | 100000 | 202 | | 255,07 | 19993 | 25922 |
| 311594 B 1:10 (4) | 0,5 | 10 | 100000 | 297 | <2 | 265,08 | 28919 | 38011 |
| Puffer PBS C 1:5 (5) | 0,5 | 5 | 100000 | 0 | | 265,09 | 29499 | 1931 |
| Puffer PBS C 1:5 (5) | 0,5 | 5 | 100000 | 2 | | 265,10 | 30666 | 2641 |
| Puffer PBS C 1:10 (6) | 0,5 | 10 | 100000 | <2 | | 265,11 | 18919 | 1824 |
| Puffer PBS C 1:10 (6) | 0,5 | 10 | 100000 | <2 | | 205,12 | 10812 | 2072 |
| 311594 D 1:5 (7) | 0,5 | 5 | 100000 | 821 | 572 | 265,13 | 13639 | 72060 |
| 311594 D 1:5 (7) | 0,5 | 5 | 100000 | 820 | | 266,14 | 10835 | 73990 |
| 311594 D 1:10 (8) | 0,5 | 10 | 100000 | 407 | | 265,15 | 40842 | 72605 |
| 311594 D 1:10 (8) | 0,5 | 10 | 100000 | 440 | | 265,16 | 30240 | 57937 |
| TKE 1:5 (9) | 0,5 | 5 | 100000 | -1 | 49,75 | 266,17 | -1 | -1 |
| TKE 1:5 (9) | 0,5 | 5 | 100000 | 16 | | 265,18 | 36970 | 7232 |
| TKE 1:10 (10) | 0,5 | 10 | 100000 | 17 | | 265,19 | 45360 | 6403 |
| TKE 1:10 (10) | 0,5 | 10 | 100000 | 160 | | 285,20 | 30372 | 23400 |
| 311594 F 1:5 (11) | 0,5 | 5 | 100000 | 393 | 400,25 | 265,21 | 27779 | 93765 |
| 311594 F 1:5 (11) | 0,5 | 5 | 100000 | 359 | | 265,22 | 27093 | 03334 |
| 311594 F 1:10 (12) | 0,5 | 10 | 100000 | 403 | | 205,23 | 39560 | 69866 |
| 311594 F 1:10 (12) | 0,5 | 10 | 100000 | 473 | | 265,24 | 30846 | 64072 |
| H2O (13) | 0,5 | - | 100000 | 0 | | 285,25 | 61656 | -1 |
| H2O (13) | 0,5 | - | 100000 | 0 | | 265,26 | 47800 | -1 |
| H2O (14) | 0,5 | - | 100000 | 0 | | 265,27 | 24046 | 1739 |
| H2O (14) | 0,5 | - | 100000 | 0 | | 265,20 | 43898 | 2406 |
| H2O (15) | 0,5 | - | 100000 | 0 | | 265,28 | 47080 | 3223 |
| H2O (15) | 0,5 | - | 100000 | 0 | | 265,30 | 28895 | 1978 |
| 20ng DNA (16) | 0,5 | - | 100000 | 22 | | 265,31 | 47115 | 40101 |
| 20pg DNA (16) | 0,5 | - | 100000 | 22 | | 265,32 | 43085 | 44121 |
| 5pg | 0,5 | - | 100000 | 4,3 | | 265,33 | 11919 | 44507 |
| 5pg | 0,5 | - | 100000 | 3,6 | | 265,34 | 12681 | 39280 |
| PCR | 0,5 | - | 100000 | 0 | | 205,35 | -1 | -1 |
| PCR | 0,5 | - | 100000 | 0 | | 265,36 | -1 | -1 |

In Fig.2 ist die graphische Auswertung der quantitativen Bestimmung der PCR-Produkte dargestellt. In den verschiedenen Bahnen sind die Intensitäten der Fluoreszenzsignale der PCR-Produkte (und Nebenprodukte) dargestellt. Die Produkte sind anhand ihrer definierten Größe (in bp) identifizierbar. Der Standard pAlu20 (S) erscheint bei 126 bp, der Wildtyp-Peak (P) bei 146 bp Peak-flächen und Berechnung der DNA-Menge sind in Tabelle 2 dargestellt.

3. Quantifizierung chromosomaler Hühner-DNA

Sinn dieser Optimierung von Primern und PCR-Bedingungen ist die genaue und spezifische Messung im Konzentrationsbereich 1 pg - 100 pg Hühner-DNA pro ml Probe. Um die gewünschte Spezifität und eine Sensitivität der Messung im pg-Bereich zu erreichen, wurde als Zielsequenz für die PCR die Sequenz einer repetitiven DNA-Familie ausgewählt. Diese DNA-Familien sind nicht nur zum Großteil artspezifisch, sondern treten in großer Kopienzahl im Genom auf und erfüllen deshalb die Kriterien für eine Bestimmung von geringen Mengen spezifischer DNA. In der Familie der Aves (Vögel) wurde die CR1-repetitive DNA-Familie beschrieben, die in 7000 bis 20000 Kopien pro Genom vorhanden sein sollen (Stumph et al., Nucleic Acids Res.9 (1981) 5383-5397). Ein Vergleich von verschiedenen Mitgliedern dieser Familie zeigt eine hochkonservierte Region an der Stelle 261 bis 391 (nach der Numerierung von Stumph et al. 1984). Das Primerpaar zur Bestimmung der Hühner-DNA wurde durch Alignment der repetitiven Sequenzen so ausgewählt, daß es spezifisch innerhalb dieser konservierten Sequenz bindet und ein 84 bp langes DNA-Fragment amplifiziert, nämlich

```
CR1:      ATGAGGCACTGGAACAGGTTGCCC bp 260~283  Seq.ID 3.


CR1A      CAGGGCCACATCCAGCCTGG     bp 345-326  Seq.ID 4
```

(Numerierung nach Stumph et al. (1984)). Die Primer wurden unter Verwendung der Phosphoamidit-Chemie auf einem DNA Synthesizer hergestellt (Applied Biosystems 394 DNA Synthesizer)

Die Standard-Plasmide pCR1+11 und pCR1-8 wurden durch Insertion synthetischer Oligonukleotide, welche die von CR1 stammende Sequenz von der Position 260 bis 345 (gemäß Stumph et al.) enthielten, zwischen die NcoI- und SacI-Stelle des pBluescript-Vektors erhalten . Für pCR1+11 enthält die CR1-Sequenz ein Insert von 11 Nukleotiden, und für das Standard-Plasmid pCR1-8 eine Deletion von 8 Nukleotiden an der Position 302 (gemäß Stumph et al.). Daher haben die von diesen Standard-Plasmiden stammenden PCR-Produkte eine Länge von 95 bzw. 76 bp. Das Plasmid wurde gereinigt (QUIAGEN-Verfahren), die Konzentration durch spektroskopische Messung bei 260 nm bestimmt, mit EcoRI geschnitten und in einem 10mM TRIS/HCl pH 8/0,1 mM EDTA-Puffer verdünnt (Sambrook et al. Molecular Cloning, Second Edition, Cold Spring Harbor Lab Press, Cold Spring Harbor (1989)).

Die Erstellung der Eichgeraden erfolgt in analoger Weise zu 2.1. Im Experiment wurden 200, 40, 20, 10, 4 und 2 pg genomischer DNA aus Hühnerzellen (CEF-Zellen) extrahiert und einer PCR mit den fluoreszenz-markierten Primern CR1 und CR1A unterzogen. Als Spezifitätskontrolle wurden 100 pg Maus- und menschliche chromosomale DNA ebenfalls der PCR unterzogen. Als Negativkontrolle dienten 10 µl Wasser.

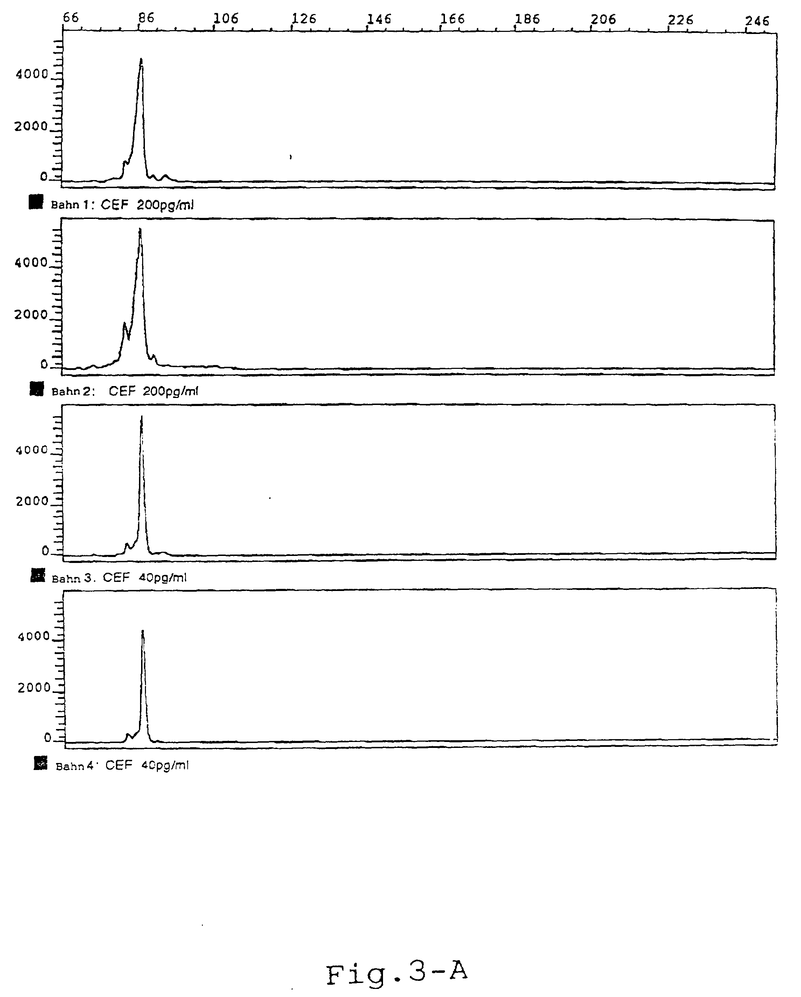

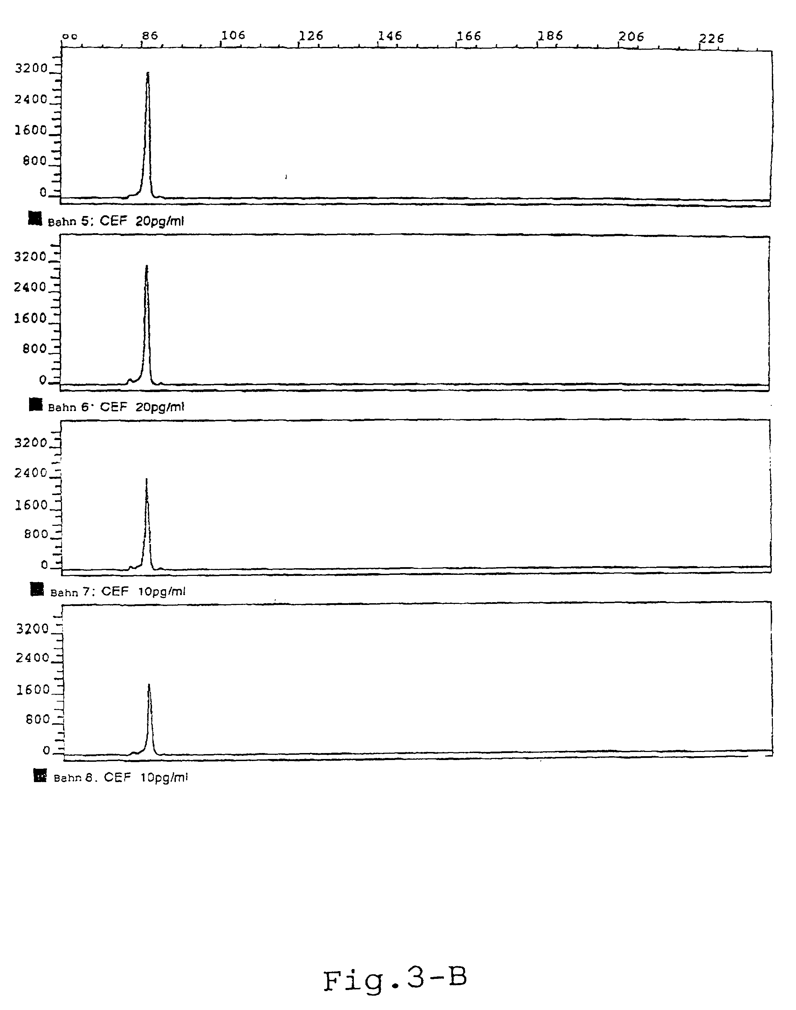

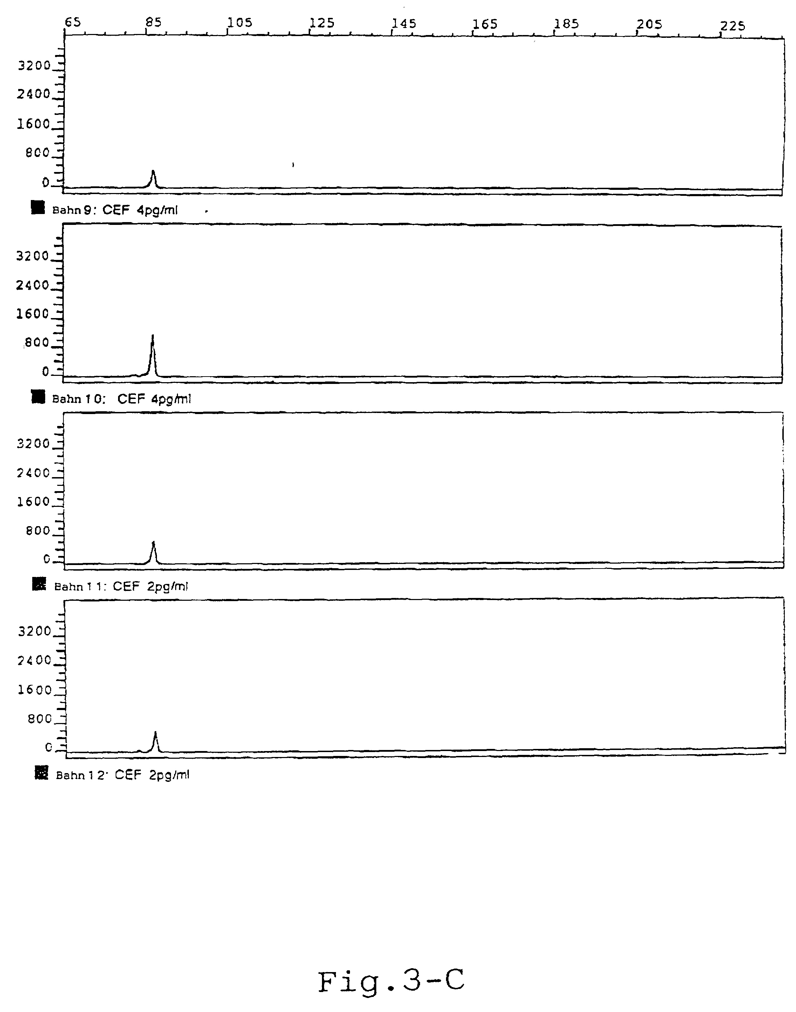

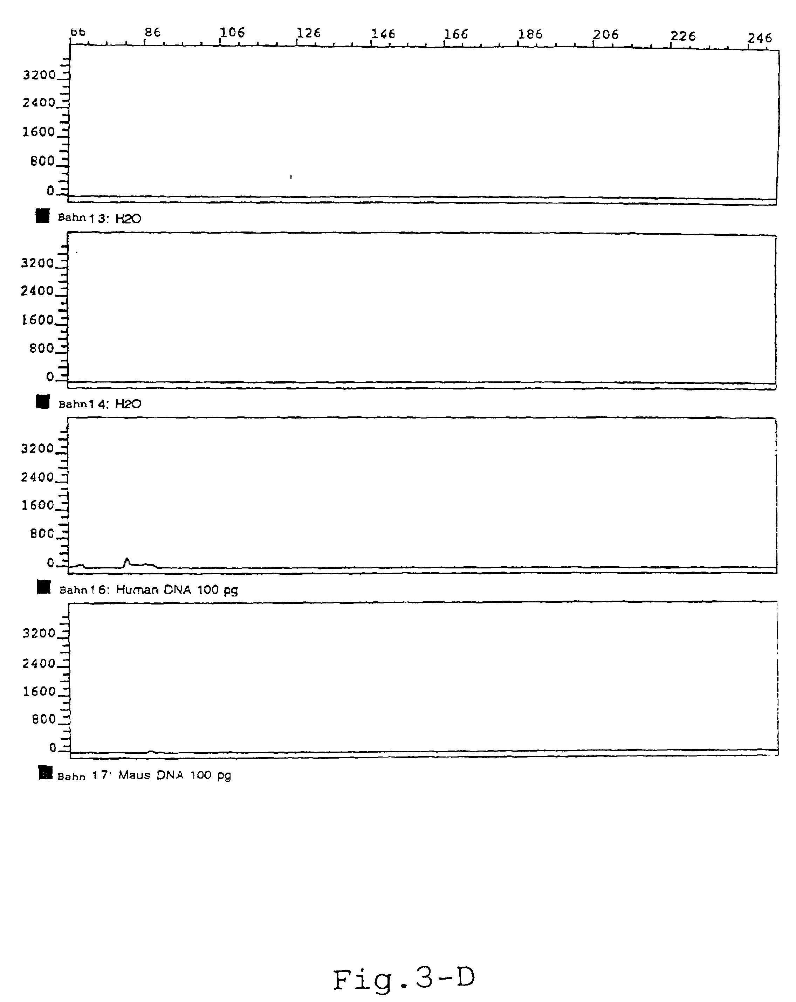

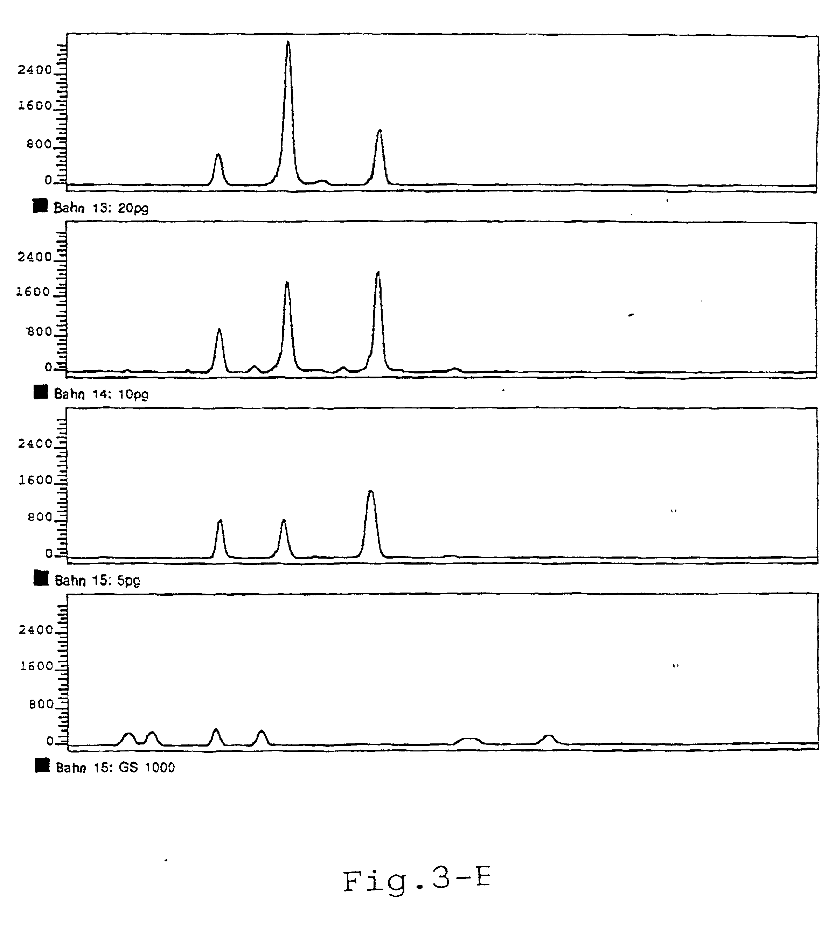

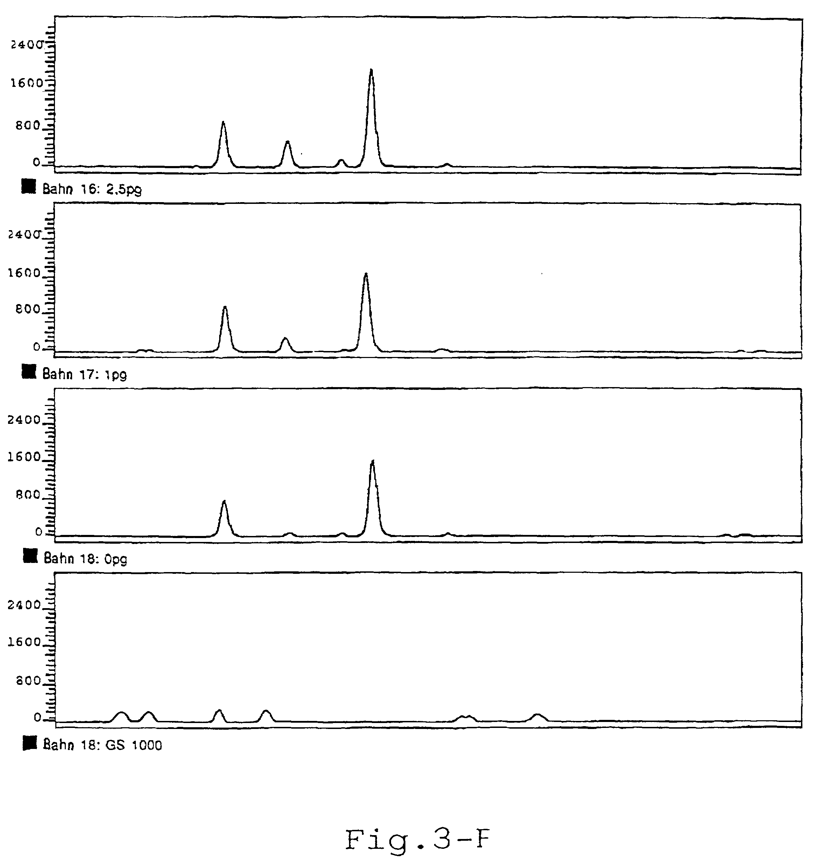

Die Ergebnisse der Untersuchungen sind in Tabelle 3 und Fig.3-A bis 3-D dargestellt.

Tabelle 3 gibt Art, Umfang und Meßwerte der gemessenen Proben wieder. In Spalte 1 ist die Nummer der Messung (entspricht einer Bahn-Nummer in Fig.3) angegeben; Spalte 2 gibt die Menge an eingesetzter genomischer DNA von CEF-Zellen an; Spalte 3 gibt die Fläche des gemessenen Peaks an.

TABELLE 3

| | | |
|---|---|---|
| | | |
| 1 | CEF 200 pg/ml | 61192 |
| 2 | CEF 200 pg/ml | 77128 |
| 3 | CEF 40 pg/ml | 36856 |
| 4 | CEF 40 pg/ml | 29229 |
| 5 | CEF20 pg/ml | 22035 |
| 6 | CEF20 pg/ml | 19008 |

TABELLE 3   (fortgesetzt)

| 7 | CEF 10 pg/ml | 13658 |
|---|---|---|
| 8 | CEF 10 pg/ml | 11333 |
| 9 | CEF 4 pg/ml | 2958 |
| 10 | CEF 4 pg/ml | 6533 |
| 11 | CEF 2 pg/ml | 3471 |
| 12 | CEF 2 pg/ml | 2907 |
| 13 | H2O | 0 |
| 14 | H2O | 0 |
| 16 | Human DNA 100 pg/ml | 0 |
| 17 | Maus DNA 100 pg/ml | 0 |
|  |  |  |

In Fig.3 ist die graphische Auswertung der Bestimmung der PCR-Produkte dargestellt. In den verschiedenen Bahnen sind die Intensitäten der Fluoreszenzsignale der PCR-Produkte (und Nebenprodukte) dargestellt. Die Produkte sind anhand ihrer definierten Größe (in bp) identifizierbar. Der Wildtyp-Peak tritt bei 86 bp auf, Peakflächen sind in Tabelle 3 dargestellt.

In sämtlichen Bahnen, in denen Hühner-DNA eingesetzt wurde, erscheint ein definierter Peak bei 84 bp. Im Gegensatz dazu wird keine signifikante Menge an 84 bp-spezifischen Produkt detektiert, auch wenn große Mengen an menschlicher bzw. Maus-DNA eingesetzt wurden. Aufgrund dieser Ergebnisse kann man die Nachweisgrenze von von chromosomaler Hühner-DNA im erfindungsgemäßen Verfahren auf kleiner als 2 pg/ml festlegen.

3.2. Quantifizierung von Hühner-DNA

Verdünnungen von Hühner-DNA von 25, 10, 5, 2,5 und 1 pg/ml und Wasser wurden extrahiert und der PCR unterzogen. Tabelle 4 gibt die Anzahl der zugesetzten Standard-Plasmid-pCR+11 und·Plasmid-PCR1-8 (Spalte 1 und 2), die Verdünnung (Spalte 3), das Volumen (Spalte 4), die Probenbeschreibung (Spalte 5), den Kommentar (Spalte 6), die Anzahl der CR1-Sequenzen in der Probe, berechnet mit dem -Standard (Spalte 7) oder mit dem +Standard (Spalte 8), den Mittelwert in pg von Hühner-DNA (Spalte 9), die Bahn, in welcher die Probe analysiert wurde (Spalte 10) und die Peak-Flächen von -Standard (Spalte 11), Wildtyp (Spalte 12) und +Standard (Spalte 13). Die graphische Auswertung der Bestimmung der PCR-Produkte ist in Fig. 3-E veranschaulicht. Die PCR-Produkte können durch ihre Größe identifiziert werden. Der Wildtyp-Peak erscheint bei 84 bp, der Minus-Standard-Peak bei 76 bp und der Plus-Standard bei 95 bp, die Peak-Flächen der Flureszenz-Signale sind in Tabelle 4 angegeben.

TABELLE 4

| N- zugeg. | N+ zugeg. | Verdünnung | Vol. | Probe | Erläuterung | N-Base | N+Base | Ergebnis | GSW, Bahn | A- | Awt | A+ |
|---|---|---|---|---|---|---|---|---|---|---|---|---|
| 5000 | 15000 | 1 | 0,25 | 25pg/ml | Hühner 32 | 113964 | 154065 | 26,8029 | Hühner 32,13 | 2916 | 16616 | 6471 |
| 5000 | 15000 | 1 | 0,25 | 10pg/ml | Hühner 32 | 51650 | 54126 | 10,5776 | Hühner 32,14 | 4132 | 10671 | 11829 |
| 5000 | 15000 | 1 | 0,25 | 5pg/ml | Hühner 32 | 22445 | 29066 | 5,1511 | Hühner 32,15 | 3705 | 4158 | 8583 |
| 5000 | 15000 | 1 | 0,25 | 2,5pg/ml | Hühner 32 | 13426 | 15697 | 2,9123 | Hühner 32,16 | 4342 | 2915 | 11142 |
| 5000 | 15000 | 1 | 0,25 | 1pg/ml | Hühner 32 | 6536 | 8486 | 1,5022 | Hühner 32,17 | 4461 | 1458 | 10308 |
| 5000 | 15000 | 1 | 0,25 | 0pg/ml | Hühner 32 | -1 | -1 | 0 | Hühner 32,18 | 3560 | -1 | 9069 |

**Patentansprüche**

1. Verfahren zur Quantifizierung von genomischer Gesamt-DNA in einer Probe, umfassend die Schritte:

   - Zugabe einer gegebenen Menge mindestens einer bekannten Nukleinsäure als interner Standard zur Probe, wobei sich die Standard-Nukleinsäure(n) zumindestens in einem zu detektierenden Merkmal von der zu quantifizierenden genomischen DNA unterscheidet;
   - Amplifizieren der genomischen DNA und der Standard-Nukleinsäure in der Probe durch ein Nukleinsäure-Amplifikationsverfahren unter Verwendung von Primern, die zu repetitiven genemischen Sequenzen komplementär sind; und
   - Bestimmung der Menge an amplifizierter repetitiver Sequenz und der Menge an amplifizierter Standard-Nukleinsäure(n) und ausgehend von der (den) erhaltenen Menge(n) an Standardard-Nukleinsäure(n) Bestimmung der Menge der ursprünglich in der Probe vorhandenen genomischen DNA.

2. Verfahren nach Anspruch 1 oder 2, **dadurch gekennzeichnet, dass** die Primer an eine Alu-Sequenz oder Alu-äquivalente Sequenz binden.

3. Verfahren nach Anspruch 1, **dadurch gekennzeichnet, dass** die Primer zu einem Teil der Alu-äquivalenten Konsensussequenz komplementär sind.

4. Verfahren nach Anspruch 3, **dadurch gekennzeichnet, dass** die Alu-äquivalenten Konsensussequenz aus Wirbeltieren, wie Nagetieren oder Primaten, ist.

5. Verfahren nach einem der Ansprüche 1 bis 4, **dadurch gekennzeichnet, dass** beim Amplifizieren Primer mit fluoreszierenden, radioaktiven oder chemischen Gruppen, die mit affinen Proteinen und nachgeschaltetenen Detektionsreaktionen detektiert werden können, verwendet werden.

6. Verfahren nach einem der Ansprüche 1 bis 5, **dadurch gekennzeichnet, dass** die Amplifikation in der exponentiellen Phase gestoppt wird.

7. Verfahren nach einem der Ansprüche 1 bis 6, **dadurch gekennzeichnet, dass** die Bestimmung der Menge an amplifizierten Nukleinsäure unter Verwendung eines Nukleinsäure-Detektionsgerätes erfolgt.

8. Verfahren nach Anspruch 7, **dadurch gekennzeichnet, dass** das Nukleinsäure-Detektionsgerät ein fluoreszenzempfindliches Detektionsgerät ist.

9. Verfahren nach einem der Ansprüche 1 bis 8, **dadurch gekennzeichnet, dass** kontaminierende genomische DNA bestimmt wird.

10. Verfahren nach einem der Ansprüche 1 bis 9, **dadurch gekennzeichnet, dass** die genomische DNA von CHO-, Vero-, BHK-, SK-Hepl-, Hybridom- oder CEC-Zellen quantifiziert wird.

11. Verfahren nach einem der Ansprüche 1 bis 10, **dadurch gekennzeichnet, dass** von der Menge an amplifizierter Standard-Nukleinsäure mittels einer Eichgerade die Menge der in der Probe zu quantifizierenden genomischen DNA bestimmt wird.

12. Verwendung eines Verfahrens nach einem der Ansprüche 1 bis 11 zur Überprüfung und Qualitätskontrolle von biologischen Produkten.

13. Primer mit der Sequenz gemäß der SEQ ID. 1, 2, 3, und 4.

14. Standardplasmide umfassend das Plasmid mit der Bezeichnung pAlu-wt bestehend aus dem Plasmid pCRII enthaltend die Alu repeat-spezifische Sequenz von Basenpaar 148 bis 294; das Plasmid mit der Bezeichnung pAlu20, abgeleitet von Plasmid pAlu-wt mit einer Deletion von 20 Basenpaaren bei Basenpaar 178 der Alu-Sequenz; das Plasmid mit der Bezeichnung pCR1-wt bestehend aus dem Plasmid pCRII enthaltend die CR1-Sequenz von Basenpaar 260 bis 345; das Plasmid mit der Bezeichnung pCR1+11 abgeleitet von Plasmid pCR1-wt mit einer Insertion von 11 Basenpaaren bei Basenpaar 300; und das Plasmid mit der Bezeichnung pCR1-8 abgeleitet von Plasmid pCR1-wt mit einer Deletion bei Basenpaar 302.

**15.** Set zur Quantifizierung genomischer Nukleinsäure in einer Probe, welches umfasst:

- mindestens eine bekannte Nukleinsäure als internen Standard, welche sich von der zu quantifizierenden Nukleinsäure in mindestens einem nachweisbaren Charakteristikum unterscheidet,
- Fluoreszenz-markierte Primer, die an repetitive genomische Sequenzen binden,
- positive Kontrollen, welche bekannte Mengen genomischer Nukleinsäure aufweisen,
- eine negative Kontrolle, welche einen von Nukleinsäuren freien Puffer aufweist, und
- eine Arbeitsanleitung.

**16.** Set zur Quantifizierung genomischer Nukleinsäure in einer Probe, welches umfasst:

- als internen Standard das Plasmid mit der Bezeichnung pAlu20 mit einer Deletion von 20 Basenpaaren bei Basenpaar 178 der Alu-repeat-spezifischen Sequenz, abgeleitet von Plasmid pAlu-wt bestehend aus dem Plasmid pCRII enthaltend die Alu-repeat-spezifische Sequenz von Basenpaar 148 bis 294,
- die Fluoreszenz-markierten Primer mit der Sequenz gemäß SEQ.ID. NO. 1 und SEQ.ID. No. 2,
- positive Kontrolle(n), die bekannte Menge(n) an Vero-Zell-DNA aufweist(en),
- eine negative Kontrolle, welche einen von Nukleinsäuren freien Puffer aufweist, und
- eine Arbeitsanleitung.

**17.** Set zur Quantifizierung genomischer Nukleinsäure einer Probe, welches umfasst:

- als internen Standard, die Plasmide mit der Bezeichnung pCR1+11 abgeleitet von Plasmid pCR1-wt mit einer Insertion von 11 Basenpaaren bei Basenpaar 300 und das Plasmid mit der Bezeichnung pCR1-8 abgeleitet von Plasmid pCR2-wt mit einer Deletion bei Basenpaar 302, wobei das Plasmid mit der Bezeichnung pCR1-wt aus dem Plasmid pCRII enthaltend die CR1-Sequenz von Basenpaar 260 bis 345 besteht,
- Floureszenz-markierte Primer mit der Sequenz gemäß SEQ.ID. NO. 3 und SEQ.ID. No. 4,
- positive Kontrolle(n), die bekannte Menge(n) and Vogel-DNA aufweist(en),
- eine negative Kontrolle, welche einen von Nukleinsäuren freien Puffer aufweist, und
- eine Arbeitsanleitung.

**Claims**

1. A method for quantifying genomic total DNA in a sample, which method comprises the steps of:

- adding to the sample a given amount of at least one known nucleic acid as an internal standard, said standard nucleic acid(s) differing from the genomic DNA to be quantified in at least one characteristic feature to be detected;
- amplifying the genomic DNA and the standard nucleic acid within the sample by a nucleic acid amplification process using primers that are complementary to repetitive genomic sequences; and
- determining the amount of repetitive sequence amplified and the amount of standard nucleic acid(s) amplified and, departing from the amount(s) of standard nucleic acid(s) obtained, determining the amount of the genomic DNA initially contained in the sample.

2. A method according to claim 1, **characterized in that** the primers bind to an Alu sequence or Alu-equivalent sequence.

3. A method according to claim 1, **characterized in that** the primers are complementary to a part of the Alu-equivalent consensus sequence.

4. A method according to claim 3, **characterized in that** the Alu-equivalent consensus sequence is derived from vertebrates such as rodents or primates.

5. A method according to any one of claims 1 to 4, **characterized in that** primers including fluorescent, radioactive or chemical groups capable of being detected by affine proteins and consecutive detection reactions are used for amplification.

6. A method according to any one of claims 1 to 5, **characterized in that** amplification is stopped in the exponential phase.

7. A method according to any one of claims 1 to 6, **characterized in that** the determination of the amount of amplified nucleic acid is effected using a nucleic acid detection device.

8. A method according to claim 7, **characterized in that** the nucleic acid detection device is a fluorescence-sensitive detection device.

9. A method according to any one of claims 1 to 8, **characterized in that** contaminating genomic DNA is determined.

10. A method according to any one of claims I to 9, **characterized in that** the genomic DNA of CHO, Vero, BHK, SK-Hepl, hybridoma or CEC cells is quantified.

11. A method according to any one of claims 1 to 10, **characterized in that** the amount of genomic DNA to be quantified in the sample is determined from the amount of amplified standard nucleic acid by means of a straight calibration line.

12. The use of a method according to any one of claims 1 to 11 for the examination and quality assessment of biological products.

13. Primers having the sequence according to SEQ ID. 1, 2, 3 and 4.

14. Standard plasmides comprising the plasmid having the designation pAlu-wt, which consists of the plasmid pCRII containing the Alu-repeat-specific sequence of base pairs 148 to 294; the plasmid having the designation pAlu20, which is derived from the plasmid pAlu-wt with a deletion of 20 base pairs at base pair 178 of the Alu sequence; the plasmid having the designation pCR1-wt, which consists of the plasmid pCRII containing the CR1 sequence of base pairs 260 to 345; the plasmid having the designation pCR1+11, which is derived from the plasmid pCR1-wt with an insertion of 11 base pairs at base pair 300; and the plasmid having the designation pCR1-8, which is derived from the plasmid pCR1-wt with a deletion at base pair 302.

15. A kit to be used for the quantification of genomic nucleic acid in a sample, which kit comprises:

   • at least one known nucleic acid as an internal standard, which nucleic acid differs from the nucleic acid to be quantified in at least one detectable characteristic feature,
   • fluorescence-labelled primers that bind to repetitive genomic sequences,
   • positive controls comprising known amounts of genomic nucleic acid,
   • a negative control comprising a buffer free of nucleic acids, and
   • a working instruction.

16. A kit to be used for the quantification of genomic nucleic acid in a sample, which kit comprises:

   • as an internal standard the plasmid having the designation pAlu20 with a deletion of 20 base pairs at base pair 178 of the Alu-repeat-specific sequence, which is derived from the plasmid pAlu-wt consisting of the plasmid pCRII containing the Alu-repeat-specific sequence of base pairs 148 to 294,
   • the fluorescence-labelled primers having the sequences according to SEQ.ID.NO. I and SEQ.ID.No.2,
   • positive control(s) comprising known amount(s) of Vero cell DNA,
   • a negative control comprising a buffer free of nucleic acids, and
   • a working instruction.

17. A kit to be used for the quantification of genomic nucleic acid in a sample, which kit comprises:

   • as an internal standard the plasmids having the designation pCR1+11, which is derived from the plasmid pCR1-wt with an insertion of 11 base pairs at base pair 300, and the plasmid having the designation pCR1-8, which is derived from the plasmid pCR1-wt with a deletion at base pair 302, the plasmid having the designation pCR1-wt consisting of the plasmid pCRII containing the CR1 sequence of base pairs 260 to 345,
   • fluorescence-labelled primers having the sequences according to SEQ.ID.NO. 3 and SEQ.ID.No.4,
   • positive control(s) comprising known amount(s) of avian DNA,
   • a negative control comprising a buffer free of nucleic acids, and

- a working instruction.

**Revendications**

1. Procédé pour quantifier l'ADN génomique total d'un échantillon, qui comprend les étapes suivantes :

   - addition, à l'échantillon, d'une quantité donnée d'au moins un acide nucléique connu, servant d'étalon interne, le ou les acides nucléiques étalons se distinguant de l'ADN génomique devant être quantifié par au moins une caractéristique à détecter ;
   - amplification de l'ADN génomique et de l'acide nucléique étalon se trouvant dans l'échantillon, par une opération d'amplification d'acides nucléiques par utilisation d'amorces qui sont complémentaires de séquences génomiques répétitives ; et
   - détermination de la quantité de la séquence répétitive amplifiée et de la quantité du ou des acides nucléiques étalons amplifiés et, à partir de la ou des quantités obtenues du ou des acides nucléiques étalons, détermination de la quantité de l'ADN génomique initialement présent dans l'échantillon.

2. Procédé selon la revendication 1 ou 2, **caractérisé en ce que** les amorces se lient à une séquence Alu ou à une séquence équivalente à Alu.

3. Procédé selon la revendication 1, **caractérisé en ce que** les amorces sont complémentaires d'une partie de la séquence consensus équivalente à Alu.

4. Procédé selon la revendication 3, **caractérisé en ce que** la séquence consensus équivalente à Alu provient de vertébrés tels que les rongeurs ou les primates.

5. Procédé selon l'une des revendications 1 à 4, **caractérisé en ce que**, lors de l'amplification, on utilise des amorces comportant des groupes fluorescents, radioactifs ou chimiques, qui peuvent être détectés avec des protéines affines, puis par des réactions de détection.

6. Procédé selon l'une des revendications 1 à 5, **caractérisé en ce que** l'amplification est interrompue dans sa phase exponentielle.

7. Procédé selon l'une des revendications 1 à 6, **caractérisé en ce que** la détermination de la quantité d'acide nucléique amplifié s'effectue par utilisation d'un appareil de détection des acides nucléiques.

8. Procédé selon la revendication 7, **caractérisé en ce que** l'appareil de détection des acides nucléiques est un appareil de détection sensible à la fluorescence.

9. Procédé selon l'une des revendications 1 à 8, **caractérisé en ce que** l'on détermine l'ADN génomique contaminant.

10. Procédé selon l'une des revendications 1 à 9, **caractérisé en ce que** c'est l'ADN génomique de cellules CHO, Vero, BHK, SK-Hepl, d'hybridome ou CEC, qui est quantifié.

11. Procédé selon l'une des revendications 1 à 10, **caractérisé en ce que**, sur la quantité d'acide nucléique étalon amplifié, on détermine à l'aide d'une droite d'étalonnage la quantité de l'ADN génomique à quantifier dans l'échantillon.

12. Utilisation d'un procédé selon l'une des revendications 1 à 11 pour la surveillance et le contrôle de qualité de produits biologiques.

13. Amorce ayant une séquence selon SEQ.ID. 1, 2, 3 et 4.

14. Plasmides étalons comprenant le plasmide ayant la désignation pAlu-wt, constitué du plasmide pCRII contenant la séquence spécifique du produit de répétition Alu allant des paires de bases 148 à 294 ; le plasmide ayant la désignation pAlu20, qui dérive du plasmide pAlu-wt avec une délétion de 20 paires de bases au niveau de la paire de bases 178 de la séquence Alu ; le plasmide ayant la désignation pCR1-wt, constitué du plasmide pCRII contenant la séquence CR1 allant des paires de bases 260 à 345 ; le plasmide ayant la désignation pCR1+11, qui dérive du plasmide pCR1-wt avec une insertion de 11 paires de bases au niveau de la paire de bases 300 ; et le plasmide ayant la désignation pCR1-8, qui dérive du plasmide pCR1-wt avec une délétion au niveau de la paire de bases 302.

**15.** Kit de quantification de l'acide nucléique génomique dans un échantillon, qui comporte

- au moins un acide nucléique connu, servant d'étalon interne, et qui se distingue de l'acide nucléique à quantifier par au moins une caractéristique détectable,
- des amorces marquées par fluorescence, qui se lient à des séquences génomiques répétitives,
- des témoins positifs, qui contiennent des quantités connues de l'acide nucléique génomique,
- un témoin négatif, qui comporte un tampon exempt d'acides nucléiques, et
- un mode d'emploi.

**16.** Kit de quantification de l'acide nucléique génomique dans un échantillon, qui comporte

- en tant qu'étalon interne, le plasmide ayant la désignation pAlu20 avec une délétion de 20 paires de bases au niveau de la paire de bases 178 de la séquence spécifique du produit de répétition Alu, qui dérive du plasmide pAlu-wt constitué du plasmide pCRII contenant la séquence spécifique du produit de répétition Alu allant des paires de bases 148 à 294,
- l'amorce marquée par fluorescence ayant la séquence SEQ.ID. NO. 1 et SEQ.ID. NO. 2,
- un ou plusieurs témoins positifs, qui contiennent la ou les quantités connues d'ADN de cellule Vero,
- un témoin négatif qui contient un tampon exempt d'acides nucléiques, et
- un mode d'emploi.

**17.** Kit de quantification de l'acide nucléique génomique dans un échantillon, qui comporte :

- en tant qu'étalon interne, les plasmides ayant la désignation pCR1+11, qui dérive du plasmide pCR1-wt avec une insertion de 11 paires de bases au niveau de la paire de bases 300, et le plasmide ayant la désignation pCR1-8, qui dérive du plasmide pCR1-wt avec une délétion au niveau de la paire de bases 302, le plasmide ayant la désignation pCR1-wt étant constitué du plasmide pCRII contenant la séquence CR1 allant des paires de bases 260 à 345,
- des amorces marquées par fluorescence, avec la séquence SEQ.ID. NO. 3 et SEQ.ID. NO. 4,
- un ou plusieurs témoins positifs, qui contiennent une ou des quantités connues d'ADN Vogel,
- un témoin négatif, qui contient un tampon exempt d'acides nucléiques, et
- un mode d'emploi.

Fig.1

Fig.2-A

Fig.2-B

Fig.2-C

Fig.2-D

Fig.2-E

Fig.2-F

Fig.2-G

Fig.2-H

Fig.2-I

Fig.3-A

Fig.3-B

Fig.3-C

Fig.3-D

Fig.3-E

Fig.3-F

SEQUENZPROTOKOLL

(1) ALLGEMEINE ANGABEN:

    (i) ANMELDER:
        (A) NAME: Thomas Haemmerle
        (B) STRASSE: Hauptstrasse 46
        (C) ORT: Orth/Donau
        (D) BUNDESLAND: Austria
        (E) LAND: Austria
        (F) POSTLEITZAHL: 2304

        (A) NAME: Falko-Guenther Falkner
        (B) STRASSE: Neusiedlzeile 76A
        (C) ORT: Orth/Donau
        (D) BUNDESLAND: Austria
        (E) LAND: Austria
        (F) POSTLEITZAHL: 2304

        (A) NAME: Johann Kohl
        (B) STRASSE: Schuhmeierplatz 3/8
        (C) ORT: Wien
        (D) BUNDESLAND: Austria
        (E) LAND: Austria
        (F) POSTLEITZAHL: 1160

        (A) NAME: Michele Himmelspach
        (B) STRASSE: Laxenburgerstrasse 59/13
        (C) ORT: Wien
        (D) BUNDESLAND: Austria
        (E) LAND: Austria
        (F) POSTLEITZAHL: 1100

        (A) NAME: Friedrich Dorner
        (B) STRASSE: Peterlinigasse 17
        (C) ORT: Wien
        (D) BUNDESLAND: Austria
        (E) LAND: Austria
        (F) POSTLEITZAHL: 1238

    (ii) BEZEICHNUNG DER ERFINDUNG: Verfahren zur Quantifizierung von
                      genomischer DNA

    (iii) ANZAHL DER SEQUENZEN: 4

    (iv) COMPUTER-LESBARE FASSUNG:
        (A) DATENTRÄGER: Floppy disk
        (B) COMPUTER: IBM PC compatible
        (C) BETRIEBSSYSTEM: PC-DOS/MS-DOS
        (D) SOFTWARE: PatentIn Release #1.0, Version #1.30 (EPA)

(2) ANGABEN ZU SEQ ID NO: 1:

    (i) SEQUENZKENNZEICHEN:
        (A) LÄNGE: 28 Basenpaare
        (B) ART: Nucleotid
        (C) STRANGFORM: Einzelstrang
        (D) TOPOLOGIE: linear

    (ii) ART DES MOLEKÜLS: Genom-DNA

    (xi) SEQUENZBESCHREIBUNG: SEQ ID NO: 1:

GCCGGGCGTA GTGGCGGGCG CCTGTAGT                    28

# Fig.4-A

(2) ANGABEN ZU SEQ ID NO: 2:

    (i) SEQUENZKENNZEICHEN:
        (A) LÄNGE: 28 Basenpaare
        (B) ART: Nucleotid
        (C) STRANGFORM: Einzelstrang
        (D) TOPOLOGIE: linear

    (ii) ART DES MOLEKÜLS: Genom-DNA

    (xi) SEQUENZBESCHREIBUNG: SEQ ID NO: 2:

GAGACAGAGT CTCGCTCTGT CGCCCAGG        28


(2) ANGABEN ZU SEQ ID NO: 3:

    (i) SEQUENZKENNZEICHEN:
        (A) LÄNGE: 24 Basenpaare
        (B) ART: Nucleotid
        (C) STRANGFORM: Einzelstrang
        (D) TOPOLOGIE: linear

    (ii) ART DES MOLEKÜLS: Genom-DNA

    (xi) SEQUENZBESCHREIBUNG: SEQ ID NO: 3:

ATGAGGCACT GGAACAGGTT GCCC        24


(2) ANGABEN ZU SEQ ID NO: 4:

    (i) SEQUENZKENNZEICHEN:
        (A) LÄNGE: 20 Basenpaare
        (B) ART: Nucleotid
        (C) STRANGFORM: Einzelstrang
        (D) TOPOLOGIE: linear

    (ii) ART DES MOLEKÜLS: Genom-DNA

    (xi) SEQUENZBESCHREIBUNG: SEQ ID NO: 4:

CAGGGCCACA TCCAGCCTGG        20


# Fig.4-B